# EUROPEAN PATENT SPECIFICATION

(11) **EP 2 475 388 B1**
(45) Date of publication and mention of the grant of the patent: **08.11.2017**
(21) Application number: 10815921.1
(22) Date of filing: 01.09.2010
(51) Int. Cl.: A61K 39/395, A61K 38/16, A61K 31/7088, A61P 17/00

(54) **USE OF IL-33 ANTAGONISTS TO TREAT FIBROTIC DISEASE**
VERWENDUNG VON IL-33 ANTAGONISTEN ZUR BEHANDLUNG FIBROTISCHER ERKRANKUNGEN
UTILISATION D'ANTAGONISTES DE L'IL-33 À DES FINS DE TRAITEMENT DES MALADIES FIBROTIQUES

(30) Priority: 10.09.2009 US 241324 P
(43) Date of publication of application: 18.07.2012
(73) Proprietor: Merck Sharp & Dohme Corp., Rahway, NJ 07065-0907 (US)
(72) Inventor: RANKIN, Andrew, L., Waltham Massachusetts 02452 (US); PFLANZ, Stefan, Palo Alto California 94304 (US); MUMM, John, Palo Alto California 94304 (US)
(74) Representative: Horgan, James Michael Frederic
(86) International application number: PCT/US2010/047461
(87) International publication number: WO 2011/031600

(56) References cited:
- WO-A1-2008/132709
- WO-A1-2008/144610
- WO-A2-2007/140205
- WO-A2-2008/022761
- US-A1- 2007 160 579
- US-A1- 2008 003 199
- US-B1- 7 560 530
- RANKIN ANDREW L ET AL: "IL-33 Induces IL-13-Dependent Cutaneous Fibrosis", JOURNAL OF IMMUNOLOGY, vol. 184, no. 3, February 2010 (2010-02), pages 1526-1535, XP009167831, ISSN: 0022-1767
- PFLANZ STEFAN ET AL: "II-33 induces II-13-dependent cutaneous fibrosis", INFLAMMATION RESEARCH, vol. 59, no. Suppl. 1, 2010, page S109, XP009167834, & 8TH WORLD CONGRESS ON TRAUMA, SHOCK, INFLAMMATION AND SEPSIS/23RD SIS-EUROPE CONGRESS ON SURGICAL IN; MUNICH, GERMANY; MARCH 09 -13, 2010 ISSN: 1023-3830
- MARVIE PIERRICK ET AL: "Interleukin-33 overexpression is associated with liver fibrosis in mice and humans.", JOURNAL OF CELLULAR AND MOLECULAR MEDICINE JUN 2010, vol. 14, no. 6B, 5 June 2009 (2009-06-05), pages 1726-1739, XP009167820, ISSN: 1582-4934
- LIU X ET AL: "Anti-IL-33 antibody treatment inhibits airway inflammation in a murine model of allergic asthma", BIOCHEMICAL AND BIOPHYSICAL RESEARCH COMMUNICATIONS, ACADEMIC PRESS INC. ORLANDO, FL, US, vol. 386, no. 1, 14 August 2009 (2009-08-14), pages 181-185, XP026467441, ISSN: 0006-291X, DOI: 10.1016/J.BBRC.2009.06.008 [retrieved on 2009-06-07]
- TAJIMA SHUNJI ET AL: "ST2 gene induced by type 2 helper T cell (Th2) and proinflammatory cytokine stimuli may modulate lung injury and fibrosis.", EXPERIMENTAL LUNG RESEARCH MAR 2007, vol. 33, no. 2, March 2007 (2007-03), pages 81-97, XP009167841, ISSN: 0190-2148
- SCHMITZ J ET AL: "IL-33, an interleukin-1-like cytokine that signals via the IL-1 receptor-related protein ST2 and induces T helper type 2-associated cytokines", IMMUNITY, CELL PRESS, US, vol. 23, no. 5, 1 November 2005 (2005-11-01), pages 479-490, XP002432892, ISSN: 1074-7613, DOI: 10.1016/J.IMMUNI.2005.09.015
- KONDO YUICHI ET AL: "Administration of IL-33 induces airway hyperresponsiveness and goblet cell hyperplasia in the lungs in the absence of adaptive immune system", INTERNATIONAL IMMUNOLOGY, OXFORD UNIVERSITY PRESS, GB, vol. 20, no. 6, 1 June 2008 (2008-06-01), pages 791-800, XP009167832, ISSN: 0953-8178, DOI: 10.1093/INTIMM/DXN037
- KASAIAN M T ET AL: "IL-13 as a therapeutic target for respiratory disease", BIOCHEMICAL PHARMACOLOGY, PERGAMON, OXFORD, GB, vol. 76, no. 2, 15 July 2008 (2008-07-15), pages 147-155, XP026960884, ISSN: 0006-2952 [retrieved on 2008-04-16]
- KAKKAR R ET AL: "The IL-33/ST2 pathway: Therapeutic target and novel biomarker", NATURE REVIEWS. DRUG DISCOVERY, NATURE PUBLISHING GROUP, GB, vol. 7, no. 10, 1 January 2008 (2008-01-01), pages 827-840, XP002530680, ISSN: 1474-1784, DOI: 10.1038/NRD2660
- H. HAYAKAWA ET AL: "Soluble ST2 Blocks Interleukin-33 Signaling in Allergic Airway Inflammation", JOURNAL OF BIOLOGICAL CHEMISTRY, vol. 282, no. 36, 1 January 2007 (2007-01-01), pages 26369-26380, XP55030354, ISSN: 0021-9258, DOI: 10.1074/jbc.M704916200
- SANADA SHOJI ET AL: "IL-33/ST2 is a critical cardioprotective fibroblast-cardiomyocyte signaling system activated by mechanical overload", CIRCULATION, LIPPINCOTT WILLIAMS & WILKINS, US, vol. 114, no. 18, Suppl, 1 October 2006 (2006-10-01), page 263, XP009167836, ISSN: 0009-7322
- SANADA ET AL.: 'IL-33 and ST2 comprise a critical biomechanically induced and cardioprotective signaling system.' J. CLIN. INVEST. vol. 117, no. 6, June 2007, pages 1538 - 1549, XP008152558
- CHACKERIAN ET AL.: 'IL-1 receptor accessory protein and ST2 comprise the IL-33 receptor complex.' J. IMMUNOL. vol. 178, no. 4, 15 August 2007, pages 2551 - 2555, XP008152567
- MOUSSION ET AL.: 'The IL-1-like cytokine IL-33 is constitutively expressed in the nucleus of endothelial cells and epithelial cells in vivo: a novel 'alarmin'?' PLOS ONE. vol. 3, no. 10, 06 October 2008, pages 1 - 8, XP055016644

## Description

### FIELD OF THE INVENTION

The present disclosure relates generally to methods of treating fibrotic disease involving agents that antagonize IL-33.

### BACKGROUND OF THE INVENTION

Fibrosis is characterized by the excess accumulation of extracellular matrix (ECM) components including collagen. Wynn (2008) J. Pathol. 214:199-210; Sivakumar & Das (2008) Inflamm. Res. 57:410-418. Fibrosis is thought to be a consequence of chronic tissue irritation or damage. Wynn (2008) J. Pathol. 214:199-210; Friedman (2008) Gastroenterology 134:1655-1669; Trojanowska & Varga (2007) Curr. Opin. Rheumatol. 19:568-573; Selman & Pardo (2006) Proc. Am. Thorac. Soc. 3:364-372. The progressive replacement of parenchymal tissues with ECM is observed in fibrotic diseases such as systemic sclerosis, idiopathic pulmonary fibrosis and liver cirrhosis, leading to impaired organ function. Fibrosis is estimated to contribute to nearly 45% of deaths in the developed world. However, the cellular and molecular factors that sustain the fibrotic cascade remain poorly understood.

Idiopathic pulmonary fibrosis (IPF) is a non-neoplastic pulmonary disease characterized by excessive fibrosis (formation of scar tissue) in the lung. Meltzer & Noble (2008) Orphanet J. Rare Dis. 3:8. IPF is estimated to affect five million people worldwide, with a mean age at presentation of 66 years. IPF is typically progressive, with median survival of 2 to 5 years from diagnosis. *Id.* Lung transplant is the only effective therapy presently available. *See also* Mendelian Inheritance in Man No. 178500, available via the U.S. National Institutes of Health NCBI/OMIM website. The etiology of IPF is not completely understood.

Scleroderma (also referred to as systemic sclerosis, or SSc) is an autoimmune connective tissue disease characterized by extensive fibrosis of the skin and visceral organs, as well as obliteration of the lumen of small arteries. Gabrielli et al. (2007) Curr. Op. Immunol. 19:640. Progression may lead to hidebound skin and damage to the gastrointestinal tract, lungs, heart and kidneys. An estimated 300,000 people in the United States have scleroderma, with women outnumbering men 4:1, and with average age at diagnosis in their 40s. Autoantibodies against several intracellular (nuclear) antigens are commonly observed, and although their presence is useful in diagnosis, there is as yet no direct evidence linking these antibodies to the pathogenesis of the disease. There is currently no cure for scleroderma, although treatment of its various symptoms is often available.

The need exists for improved methods for treating fibrotic diseases, such as idiopathic pulmonary fibrosis and scleroderma.

### SUMMARY OF THE INVENTION

The present disclosure meets these needs and more by providing methods of treating fibrotic disease comprising administration of an antagonist of IL-33.

In one embodiment, the fibrosis is fibrosis of an epithelial barrier tissue, such as skin, lung or gut. In various embodiments, the fibrotic disease is selected from the group consisting of eosinophilic esophagitis, hypereosinophilic syndromes (HES), Loeffler's endomyocarditis, endomyocardial fibrosis, idiopathic pulmonary fibrosis, and scleroderma. In specific embodiments, the fibrotic disease is idiopathic pulmonary fibrosis or scleroderma.

In various embodiments, the IL-33 antagonist is selected from the group consisting of an antagonist antibody (or antigen binding fragment thereof), a small molecule, a soluble ST2 polypeptide, or a nucleic acid expression inhibitor (such as an antisense nucleic acid or an siRNA molecule). In a specific embodiment, the IL-33 antagonist is an antagonist antibody, or antigen binding fragment thereof.

### BRIEF DESCRIPTION OF THE DRAWINGS

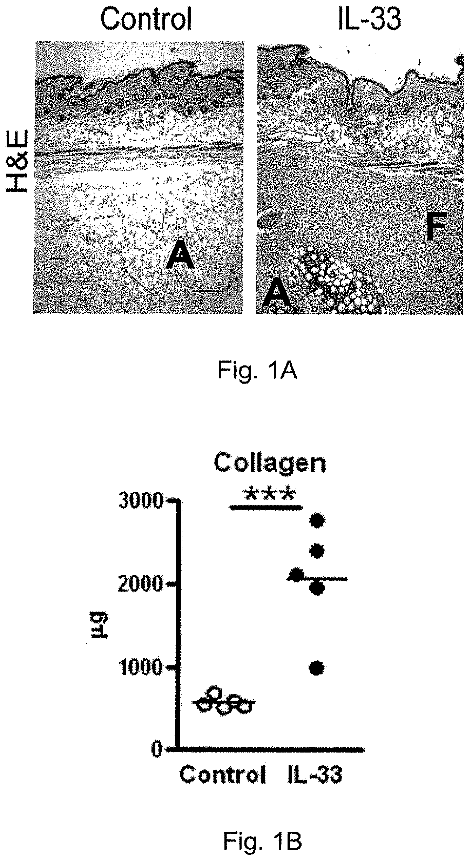

FIGS. 1A - 1D present results obtained when IL-33 is injected subcutaneously in mice, as described in greater detail at Example 3.
FIG. 1A presents photomicrographs of representative skin sections obtained from mouse serum albumin (MSA) injected- (control) or IL-33-injected (IL-33) mice stained with H&E. In this and other figures herein, the letter "A" within a photomicrograph indicates subcutaneous adipose tissue and "F" indicates a somewhat darker region of subcutaneous fibrotic tissue, which is present only in the IL-33 treated tissue. In this and other figures herein, scale bars indicate 200 µm.
FIG. 1B shows collagen content (µg) in dermal punches isolated from MSA-(control) or IL-33-injected mice. Symbols indicate values obtained from individual mice and lines indicate the average values throughout the figures. ***, p=0.001; Student's T-test.

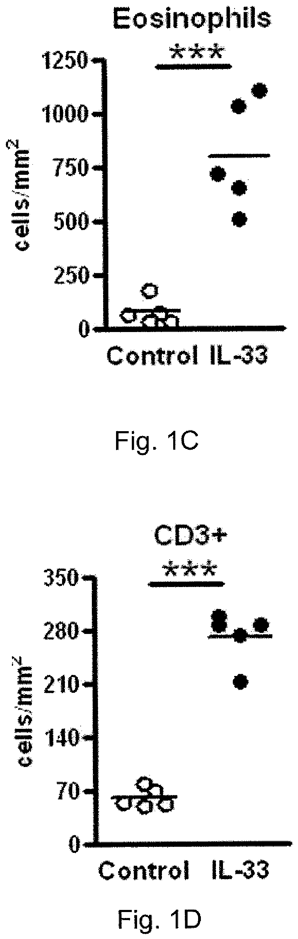

FIG. 1C shows the number of eosinophils in skin sections obtained from MSA-(open circles) or IL-33-injected (closed circles) mice. ***, p=0.001; Student's T-test.
FIG. 1D shows the number of CD3⁺ cells in skin sections obtained from MSA-(control) or IL-33-injected (closed circles) mice. Experiments were repeated independently at least two times with comparable results.

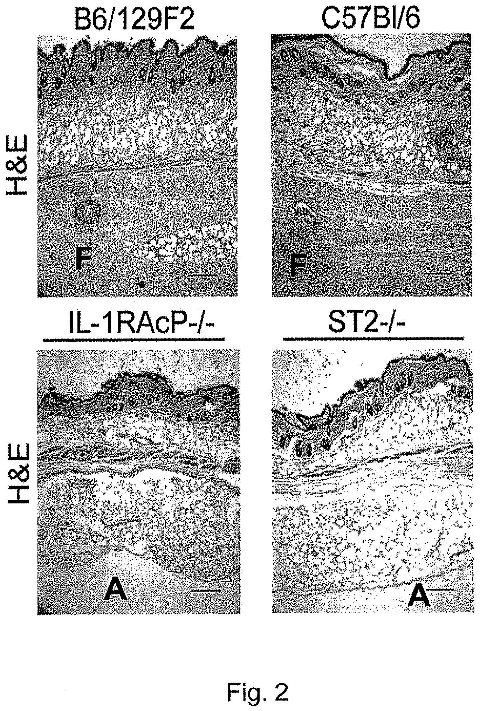

FIG. 2 presents photomicrographs of representative H&E stained skin sections obtained from wild type B6/129F2 and C57B1/6 mice (top row), as well as IL-1RAcP^{-/-}B6/129F2 and ST2^{-/-} C57B1/6 mice (bottom row), all of which had been injected with IL-33. Experiments were performed with at least 5 mice per group and were repeated independently at least two times with comparable results. *See* Example 4.

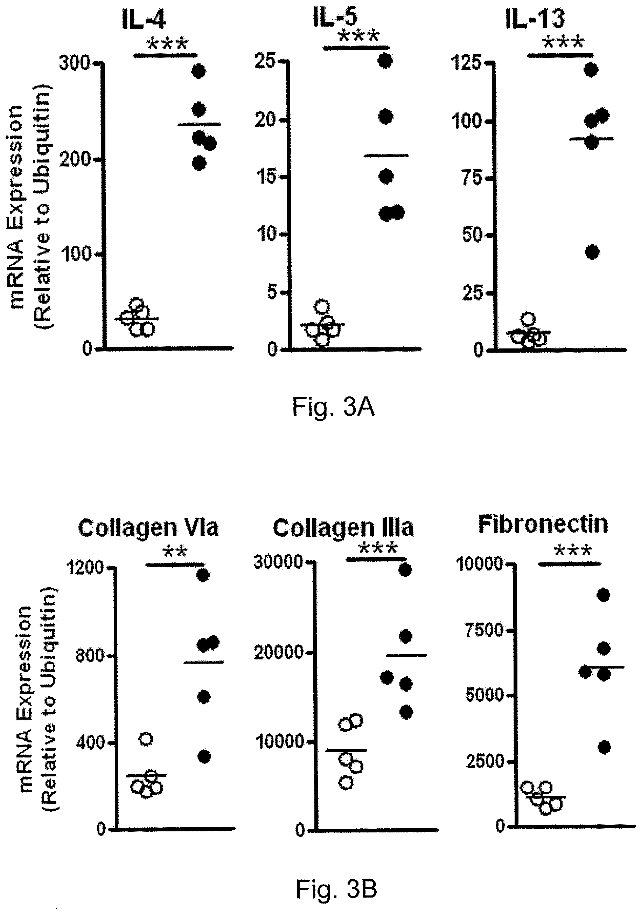

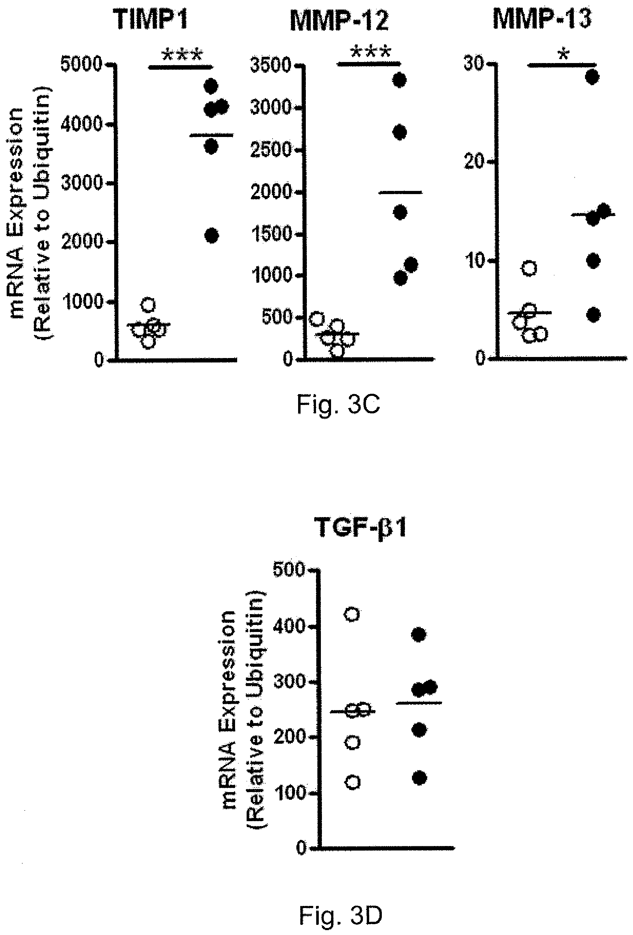

FIGS. 3A - 3D present TAQman® real time gene expression analysis of mRNA expression for various genes in skin samples obtained from MSA- (open circles) or IL-33-injected (closed circles) mice. FIG. 3A shows results for Th2 cytokines IL-4, IL-5 and IL-13. FIGS. 3B and 3C show results for various ECM-associated genes (collagen VIa, collagen IIIa, fibronectin, TIMP1, MMP-12, and MMP-13). FIG. 3D shows results for TGF-β. Individual data points represent values obtained from individual mice; lines indicate the average result. Experiments were repeated independently at least two times with comparable results. ***, p=0.008; **, p=0.016; *, p=0.032; Mann-Whitney U-test. *See* Example 5.

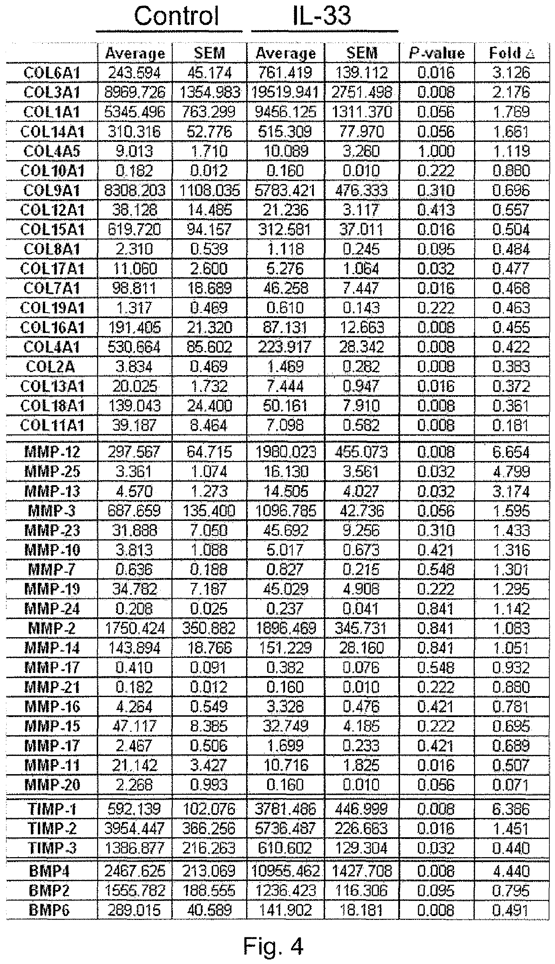

FIG. 4 provides average expression levels (and standard errors) for various extracellular matrix (ECM)-associated genes in skin samples obtained from MSA- ("Control") or IL-33-injected ("IL-33") mice (N=5 mice/condition). Expression levels were determined by TAQman® real time gene expression analysis. FIG. 4 also provides the ratio of expression in IL-33 KO cells compared with control cells ("Fold Δ"), and the P-value for the difference between IL-33 and Control expression levels calculated using the Mann-Whitney U-test.

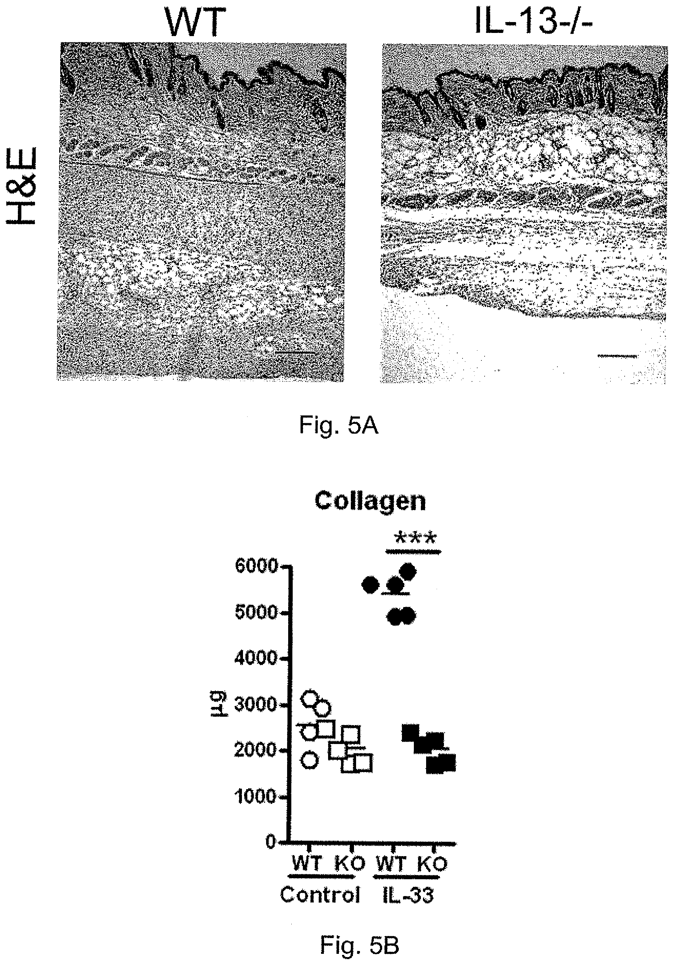

FIGS. 5A - 5G present results from experiments to assess the role of IL-13 in IL-33 responses, as described in greater detail at Example 6.
FIG. 5A presents photomicrographs of representative skin sections obtained from IL-33-injected wild-type (wt) or IL-13 KO (IL-13 ^{-/-}) mice stained with H&E.
FIG. 5B shows collagen content (µg) in dermal punches obtained from MSA-(control) and IL-33-treated (IL-33) wild type (WT) and IL-13^{-/-} (KO) mice. Symbols represent values obtained from individual mice and lines indicate the average values throughout the figure. ***, p<0.0001; Student's T-test.

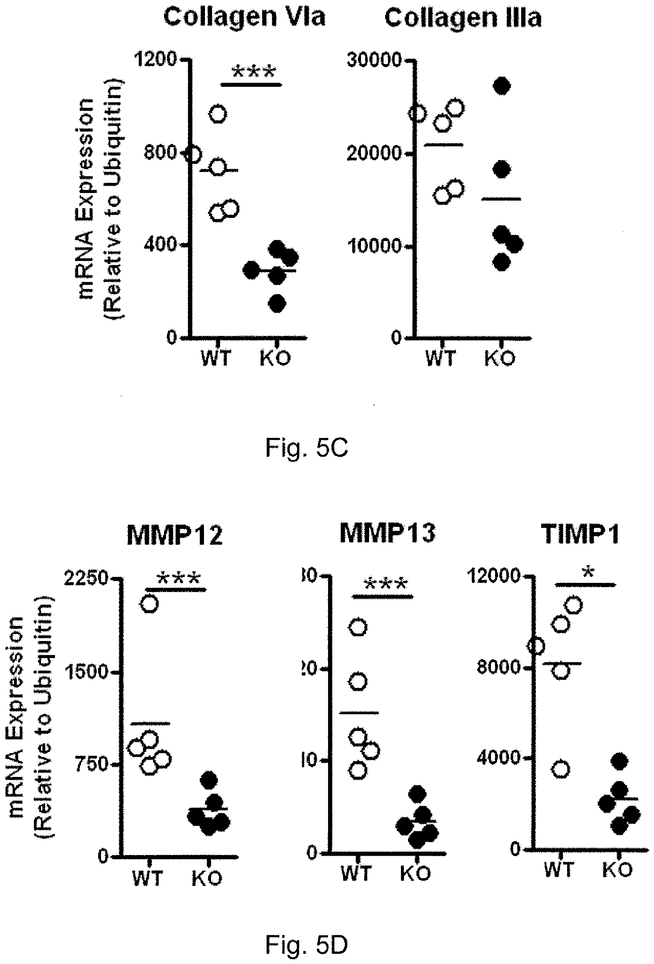

FIG. 5C shows TAQman® real time gene expression analysis of collagen VIa and collagen IIIa genes in IL-33-treated wild type (WT) and IL-13^{-/-} (KO) mice.
FIG. 5D shows TAQman® real time gene expression analysis of MMP12, MMP13 and TIMP1 genes in IL-33-treated wild type (WT) and IL-13^{-/-} (KO) mice. ***, p=0.008; *, p=0.016 Mann-Whitney U-test.

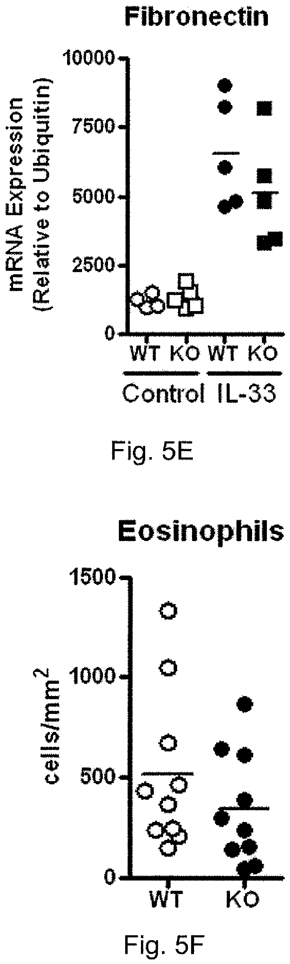

FIG. 5E shows TAQman® real time gene expression analysis of fibronectin in individual skin samples obtained from MSA- (control) or IL-33-treated wild type (WT) and IL-13^{-/-} mice (KO).
FIG. 5F shows the number of eosinophils in skin sections obtained from IL-33-injected wild type (WT) or IL-13^{-/-} (KO) mice.

### DETAILED DESCRIPTION

. GenBank accession numbers for nucleic acid and protein sequences referenced herein refer to the contents of the database as of the filing date of this application. Although such database entries may be subsequently modified, GenBank maintains a public record of all prior versions of the sequences as a function of date, making such database entries an unambiguous reference to a specific sequence.
. Citation of the references herein is not intended as an admission that the reference is pertinent prior art, nor does it constitute any admission as to the contents or date of these publications or documents.

### I. Definitions

As used herein, including the appended claims, the singular forms of words such as "a," "an," and "the," include their corresponding plural references unless the context clearly dictates otherwise.

"Approximately," as used herein, refers to the value of a parameter measured with the degree of precision that is typical in the art. Specifically, it refers to a value that would be within typical tolerances for such parameter, as understood by one of skill in the art.

As used herein, unless otherwise indicated (e.g. IL-33), the "mature" form of a protein refers to the polypeptide chain remaining after removal of the signal sequence (leader peptide), e.g. the form of the protein that it secreted from the cell. Signal sequences from the proteins disclosed herein are referenced in the Sequence Listing.

As used herein, "epithelial barrier tissue" refers to tissues that have regular contact with the external environment and provide a barrier function. Exemplary epithelial barrier tissues include skin, lung and gut.

As used herein, the term "antibody" may refer to any form of antibody that exhibits the desired biological activity. Thus, it is used in the broadest sense and specifically covers monoclonal antibodies (including full length monoclonal antibodies), polyclonal antibodies, multispecific antibodies (*e.g*., bispecific antibodies), chimeric antibodies, humanized antibodies, fully human antibodies, etc. so long as they exhibit the desired biological activity.

As used herein, when referring to antibodies, the terms "binding fragment thereof" or "antigen binding fragment thereof" encompass a fragment or a derivative of an antibody that still substantially retains the ability to bind to its target. Examples of antibody fragments include Fab, Fab', F(ab')₂, and Fv fragments; diabodies; linear antibodies; single-chain antibody molecules, e.g., sc-Fv; and multispecific antibodies formed from antibody fragments. Typically, a binding fragment or derivative retains at least 10% of its affinity for its target, e.g. no more than a 10-fold change in the dissociation equilibrium binding constant (K_{d}). Preferably, a binding fragment or derivative retains at least 25%, 50%, 60%, 70%, 80%, 90%, 95%, 99% or 100% (or more) of its binding affinity, although any binding fragment with sufficient affinity to exert the desired biological effect will be useful. It is also intended that, when specified, a binding fragment can include sequence variants with conservative amino acid substitutions.

"Specifically" or "selectively" binds, when referring to the binding of an antibody to its antigen, indicates a binding reaction which is determinative of the presence of the protein in a heterogeneous population of proteins and other biologics. As is commonly understood in the field, and reiterated here for emphasis, an antibody that is said to bind specifically to a polypeptide *comprising* a given sequence (e.g. IL-33) binds to polypeptides comprising the sequence of IL-33 but does not bind to proteins lacking the sequence of IL-33. For example, an antibody that specifically binds to a polypeptide comprising IL-33 may bind to a FLAG®-tagged form of IL-33 but will not bind to other FLAG®-tagged proteins.

The term "monoclonal antibody," as used herein, refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic epitope. In contrast, conventional (polyclonal) antibody preparations typically include a multitude of antibodies directed against (or specific for) different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al. (1975) Nature 256: 495, or may be made by recombinant DNA methods (*see, e.g.,* U.S. Pat. No. 4,816,567). The "monoclonal antibodies" may also be isolated from phage antibody libraries using the techniques described in Clackson et al. (1991) Nature 352: 624-628 and Marks et al. (1991) J. Mol. Biol. 222: 581-597, for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. U.S. Pat. No. 4,816,567; Morrison et al. (1984) Proc. Natl. Acad. Sci. USA 81: 6851-6855.

As used herein, the term "humanized antibody" refers to forms of antibodies that contain sequences from non-human (*e.g*., murine) antibodies as well as sequences from human antibodies. Such antibodies contain minimal sequence derived from non-human immunoglobulin. In general, the variable domains of a humanized antibody will comprise at least one, and up to six, of the complementarity determining regions (CDR) (also referred to as hypervariable loops) of a non-human immunoglobulin, with all or substantially all of the framework (FR) regions derived from human immunoglobulin sequences.

The term "fully human antibody" refers to an antibody that comprises human immunoglobulin protein sequences only. A fully human antibody may contain murine carbohydrate chains if produced in a mouse, in a mouse cell, or in a hybridoma derived from a mouse cell. Similarly, "mouse antibody" or "rat antibody" refer to an antibody that comprises only mouse or rat immunoglobulin sequences, respectively. A fully human antibody may be generated in a human being, in a transgenic animal having human immunoglobulin germline sequences, by phage display or other molecular biological methods.

"IL-33" refers human interleukin-33, and is also known as aka IL-1F11, NF-HEV, and C9orf26. IL-33 is further described at online Mendelian Inheritance in Man (OMIM) entry 608678 and Gene ID No. 90865, and an exemplary naturally occurring nucleic acid and protein sequences for human IL-33 are found at GenBank Accession Nos. NM_033439.2 (SEQ ID NO: 5) and AY905581 (SEQ ID NO: 6), respectively, all of which are available through the NCBI website. *See also* Schmitz et al. (2005) Immunity 23: 479-490. A mature form of IL-33 comprising residues 112 - 270 of SEQ ID NO: 6 has been proposed to be an active form of IL-33. Schmitz et al. (2005) Immunity 23:479-490.

"ST2" as used herein is synonymous with the T1/ST2 protein, and is also known as IL-1RL1, DER-4, IL-1R4, Fit-1 and IL-1RL1. ST2 is further described at OMIM entry 601203 and Gene ID No. 9173. "Soluble ST2" (sST2) refers to a soluble extracellular variant of the membrane-bound form of ST2 (ST2L). Exemplary naturally occurring sequences for human sST2 are found at GenBank Accession Nos. NM_003856.2 (SEQ ID NO: 7) / NP_003847 (SEQ ID NO: 8), and exemplary naturally occurring sequences for human ST2L are found at NM_016232.4 (SEQ ID NO: 9) / NP_057316 (SEQ ID NO: 10), all of which are available through the NCBI website. As is apparent from comparison of SEQ ID NOs: 8 and 10, sST2 comprises residues 1-323 of ST2L and an additional C-terminal five amino acid residues (SKECF).

"IL-1RAcP" means the interleukin-1 receptor accessory protein, is also known as IL-1R3 and IL1RAP. IL-1RAcP is further described at online OMIM entry 602626 and Gene ID No. 3556, and exemplary naturally occurring sequences for human IL-1RAcP are found at GenBank Accession Nos. NM_134470.2 (SEQ ID NO: 1) / NP_608273 (SEQ ID NO: 2), and at NM_002182.2 (SEQ ID NO: 3) / NP_002173 (SEQ ID NO: 4), all of which are available through the National Center for Biotechnology Information (NCBI) website.

### II. Treatment of Fibrotic Disease with IL-33 Antagonists

IL-33 is the most recently discovered member of the IL-1 cytokine family. Schmitz et al. (2005) Immunity 23:479-490. IL-33 is constitutively expressed in barrier tissues such as skin where it is found preferentially localized to the nucleus of epithelial and endothelial cells. Baekkevold et al. (2003) Am. J. Pathol. 163:69-79; Carriere et al. (2007) Proc. Nat'l Acad. Sci. (USA) 104:282-287; Kuchler et al. (2008) Am. J. Pathol. 173:1229-1242; Moussion et al. (2008) PLoS ONE 3:e3331. The receptor for IL-33 is composed of two subunits, IL-1RAcP and ST2. Schmitz et al. (2005) Immunity 23:479-490; Chackerian et al. (2007) J Immunol 179:2551-2555; U.S. Pat. No. 7,560,530.

IL-1RAcP is widely expressed, whereas ST2 expression is restricted to cell types that include Th2 cells, eosinophils, basophils, iNK:T cells and mast cells. Schmitz et al. (2005) Immunity 23:479-490; Lohning et al. (1998) Proc. Nat'l Acad. Sci. (USA) 95:6930-6935; Suzukawa et al. (2008) Lab. Invest. 88:1245-1253; Kondo et al. (2008) Int. Immunol. 20:791-800; Cherry et al. (2008) J. Allergy Clin. Immunol. 121:1484-1490; Smithgall (2008) Int. Immunol. 20:1019-1030. Consistent with the expression of ST2 by Th2-associated cell types, *in vivo* systemic administration of recombinant IL-33 induces Th2 cytokine production, eosinophilia and mucous hypersecretion in the lung and gut. Schmitz et al. (2005) Immunity 23:479-490; Kondo et al. (2008) International Immunology 20:791-800. IL-33 is released by cells undergoing necrotic cell death and in this respect IL-33 is thought to function as damage associated molecular pattern (DAMP). Cayrol & Girard (2009) Proc. Nat'l Acad. Sci. (USA) 106:9021-9026; Haraldsen et al. (2009) Trends Immunol. 30:227-233; Lüthi et al. (2009) Immunity 31: 84.

Recent studies have revealed an association between ST2 and the development of multiple diseases. A single nucleotide polymorphism in the promoter of the ST2 gene was found to associate with atopic dermatitis. Shimizu et al. (2005) Hum. Mol. Genet. 14:2919-2927. In addition, sera obtained from systemic sclerosis patients and idiopathic pulmonary fibrosis patients exhibiting acute exacerbation contained elevated levels of sST2. Kuroiwa et al. (2001) Biochem. Biophys. Res. Comm. 284:1104-1108; Tajima et al. (2003) Chest 124:1206-1214. Expression of ST2 (mRNA) was also found to be significantly increased in fibrotic liver tissue. Marvie et al. (2010) J. Cell. Mol. Med. 14(6b): 1726. Strikingly, administration of an sST2-Fc fusion protein exacerbated disease manifestations in a mouse model of liver fibrosis. Amatucci et al. (2007) J. Leukocyte Biol. 82:124; WO 2007/140205.

IL-33 has been associated with fibrotic disease. Increased expression of IL-33 message was observed in fibrotic and cirrhotic lesions adjacent to liver tumoral masses. Marvie et al. (2010) J. Cell. Mol. Med. 14(6b): 1726. In addition, administration of IL-33 limited the development of cardiac fibrosis in a mouse model of cardiac hypertrophy (Sanada et al. (2007) J. Clin. Invest. 117:1538-1549), suggesting that IL-33 could be used to treat cardiac fibrosis (U.S. Pat. App. Pub. No. 2008/0003199). Anti-IL-33 antibodies, or variants thereof, have also been proposed for the treatment of inflammatory disorders, such as asthma. WO 2008/144610.

Despite these observations, the consequences of sustained IL-33 activity in skin have not previously been reported. To determine the effects of dysregulated IL-33 signaling in skin, IL-33 was administered repeatedly subcutaneously and its effects at the injection site were monitored.

The results presented herein demonstrate that subcutaneous injection of rIL-33 can induce accumulation of extracellular matrix (collagen) and abundant inflammation comprised mainly of eosinophils, macrophages and T cells. *See* Example 3 and FIGS. 1A - 1D. These IL-33 mediated effects were ST2 and IL-1RAcP dependent (Example 4 and FIG. 2), consistent with a mechanism in which accumulation of ECM and inflammation result from IL-33 signaling via its receptor.

The pathologic changes induced by IL-33 were associated with altered expression of ECM genes in the collagen, MMP, TIMP and BMP families. *See* Example 5 and FIGS. 3A - 3D and 4. In particular, the expression levels of collagen VIa and collagen IIIa were significantly increased in skin samples obtained from IL-33 injected mice suggesting that these collagen subtypes may contribute to the increased collagen deposition in IL-33 treated skin. TIMP-1 and TIMP-2 expression were also increased by IL-33 treatment. TIMP family members can inhibit MMP-mediated collagenase activity, which may promote IL-33-induced collagen accumulation by decreasing collagen turnover. Lambert et al. (2004) Crit. Rev. Oncol. Hematol. 49:187-198.

Additional data indicate that IL-33 regulates the expression of these genes through an IL-13 dependent pathway. *See* Example 6 and FIGS. 5A - 5F. Interestingly, fibronectin-1 expression was increased independently of IL-13 in IL-33 injected skin samples, suggesting that expression of some ECM components may be regulated directly through IL-33 or other IL-13 independent downstream mediators. FIG. 5E. Overall our data highlight IL-33-mediated alterations in the expression levels of various collagen subtypes as well as enzymes known to affect ECM remodeling.

The results of the experiments presented herein establish that IL-33 induces cutaneous fibrosis and intense inflammation, and identify IL-33 as a novel factor sufficient to induce cutaneous fibrosis. These profibrotic effects were first observed in the skin of mice that received multiple subcutaneous objections of IL-33, in which the skin at the injection site thickened in IL-33 treated animals but not in controls. Experiments presented herein were then performed to further investigate the effects of dysregulated IL-33 signaling in skin. IL-33 was administered subcutaneously and its effects at the injection site were monitored. Administration of IL-33 resulted in IL-33R-dependent accumulation of eosinophils, CD3⁺ lymphocytes, F4/80⁺ mononuclear cells, increased expression of IL-13 mRNA, and the development of cutaneous fibrosis. This cutaneous fibrosis and inflammation were ST2-dependent. Consistent with extensive cutaneous tissue remodeling, IL-33 resulted in significant modulation of a number of extracellular matrix associated genes including Collagen VI, Collagen III and TIMP1. The dependence of IL-33-induced fibrosis on both IL-13 and eosinophils was established using genetically deficient mice. The data indicate that bone marrow derived eosinophils secrete IL-13 in response to IL-33 stimulation, suggesting that eosinophil derived IL-13 may promote IL-33 induced cutaneous fibrosis, i.e. that IL-13 is a critical downstream mediator of IL-33-induced cutaneous fibrosis. Collectively, these results identify IL-33 as a previously unrecognized pro-fibrotic mediator in skin and highlight the cellular and molecular pathways by which this pathology develops. The data also suggest that IL-33 may function as an endogenous DAMP involved in the initiation of fibrotic disease. *See* Rankin et al. (2010) J. Immunol. 184:1526.

IL-13 is a pro-fibrotic cytokine that is sufficient for the induction of fibrosis in skin and lung. Zheng et al. (2009) J. Invest. Dermatol. 129:742-751; Zhou et al. (1999) J. Clin. Invest. 103:779-788. In addition, IL-13 is required for the development of fibrosis in some animal models. Aliprantis et al. (2007) Proc. Nat'l Acad. Sci. (USA) 104:2827-2830; Liu et al. (2004) J. Immunol. 173:3425-3431; Kolodsick et al. (2004) J. Immunol. 172:4068-4076; Chiaramonte et al. (1999) J. Clin. Invest. 104:777-785. IL-13 has been shown to directly modulate expression of Collagen VIa, Collagen III and TIMP-1 in previous studies. Syed et al. (2005) Resp. Res. 6:9; Leonardi et al. (2003) Invest. Ophthalmol. & Visual Sci. 44:183-189. The results presented herein show that IL-33-induced fibrosis requires IL-13.

Abnormal accumulation of extracellular matrix is a defining characteristic of fibrotic diseases. Accordingly, in light of these results, neutralizing IL-33 activity, e.g. by antibody blockade, small molecule, or soluble receptor, may be efficacious in the treatment of fibrobtic diseases such as scleroderma and idiopathic pulmonary fibrosis. In addition, the prominent recruitment of eosinophils to sites of IL-33 injection suggests that eosinophil rich diseases, such as eosinophilic esophagitis and hypereosinophilic syndromes may benefit from blockade of IL-33. Blocking IL-33 activity in syndromes such as Loeffler's endomyocarditis and endomyocardial fibrosis may also be efficacious since these diseases exhibit both eosinophilia and fibrosis.

### III. IL-33 Antagonists

In various embodiments, the IL-33 antagonist of the present disclosure is an antagonist antibody (or antigen binding fragment thereof), a small molecule, a soluble ST2 polypeptide, or a nucleic acid expression inhibitor (such as an antisense nucleic acid or an siRNA molecule).

In antibody-based embodiments, the anti-IL-33 antibodies of the present disclosure comprises full length antibodies, or antigen binding fragments such as, but not limited to, Fab, Fab', Fab'-SH, Fv, scFv, F(ab')₂, and diabodies. Use of anti-IL-33 antibodies in the treatment of various inflammatory diseases, such as asthma, is disclosed at WO 2008/144610.

Any suitable method for generating monoclonal antibodies may be used. For example, a recipient may be immunized with the IL-33 or an antigenic fragment thereof. Any suitable method of immunization can be used. Such methods can include adjuvants, other immunostimulants, repeated booster immunizations, and the use of one or more immunization routes. The eliciting antigen may be a single epitope, multiple epitopes, or the entire protein alone or in combination with one or more immunogenicity enhancing agents known in the art.

Any suitable method can be used to elicit an antibody with the desired biologic property of inhibiting IL-33 activity. It is desirable to prepare monoclonal antibodies (mAbs) from various mammalian hosts, such as mice, rodents, primates, humans, etc. Techniques for preparing such monoclonal antibodies may be found in, *e.g.,* Stites et al. (eds.) BASIC AND CLINICAL IMMUNOLOGY (4th ed.) Lange Medical Publications, Los Altos, CA, and references cited therein; Harlow and Lane (1988) ANTIBODIES: A LABORATORY MANUAL CSH Press; Goding (1986) MONOCLONAL ANTIBODIES: PRINCIPLES AND PRACTICE (2d ed.) Academic Press, New York, NY. Thus, monoclonal antibodies may be obtained by a variety of techniques familiar to researchers skilled in the art. Typically, spleen cells from an animal immunized with a desired antigen are immortalized, commonly by fusion with a myeloma cell. *See* Kohler and Milstein (1976) Eur. J. Immunol. 6:511-519. Alternative methods of immortalization include transformation with Epstein Barr Virus, oncogenes, or retroviruses, or other methods known in the art. *See, e.g.,* Doyle et al. (eds. 1994 and periodic supplements) CELL AND TISSUE CULTURE: LABORATORY PROCEDURES, John Wiley and Sons, New York, NY. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according, *e.g*., to the general protocol outlined by Huse et al. (1989) Science 246:1275-1281.

Other suitable techniques involve selection of libraries of antibodies in phage or similar vectors. *See, e.g.,* Huse et al. (1989) Science 246:1275; and Ward et al. (1989) Nature 341:544. The polypeptides and antibodies of the present disclosure may be used with or without modification, including chimeric or humanized antibodies. Also, recombinant immunoglobulins may be produced, *see* Cabilly U.S. Patent No. 4,816,567; and Queen et al. (1989) Proc. Nat'l Acad. Sci. USA 86:10029-10033; or made in transgenic mice, see Mendez et al. (1997) Nature Genetics 15:146-156; also see Abgenix and Medarex® technologies.

Also contemplated are chimeric antibodies. As noted above, typical chimeric antibodies comprise a portion of the heavy and/or light chain identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al. (1984) Proc. Natl. Acad. Sci. USA 81: 6851-6855).

Antagonists of IL-33 signaling also include antisense and interfering nucleic acid molecules, such as siRNA molecules. Antisense and siRNA molecules that reduce the expression of IL-33 may be designed based on the coding sequence for IL-33, as disclosed herein at SEQ ID NO: 5. Methods of producing and using siRNA are disclosed, e.g., at U.S. Pat. Nos. 6,506,559 (WO 99/32619); 6,673,611 (WO 99/054459); 7,078,196 (WO 01/75164); 7,071,311 and PCT publications WO 03/70914; WO 03/70918; WO 03/70966; WO 03/74654; WO 04/14312; WO 04/13280; WO 04/13355; WO 04/58940; WO 04/93788; WO 05/19453; WO 05/44981; WO 03/78097 (U.S. patents are listed with related PCT publications). Exemplary methods of using siRNA in gene silencing and therapeutic treatment are disclosed at PCT publications WO 02/096927 (VEGF and VEGF receptor); WO 03/70742 (telomerase); WO 03/70886 (protein tyrosine phosphatase type IVA (Prl3)); WO 03/70888 (Chk1); WO 03/70895 and WO 05/03350 (Alzheimer's disease); WO 03/70983 (protein kinase C alpha); WO 03/72590 (Map kinases); WO 03/72705 (cyclin D); WO 05/45034 (Parkinson's disease). Exemplary experiments relating to therapeutic uses of siRNA have also been disclosed at Zender et al. (2003) Proc. Nat'l. Acad. Sci. (USA) 100:7797; Paddison et al. (2002) Proc. Nat'l. Acad. Sci. (USA) 99:1443; and Sah (2006) Life Sci. 79:1773. siRNA molecules are also being used in clinical trials, e.g., of chronic myeloid leukemia (CML) (ClinicalTrials.gov Identifier: NCT00257647) and age-related macular degeneration (AMD) (ClinicalTrials.gov Identifier: NCT00363714).

Although the term "siRNA" is used herein to refer to molecules used to induce gene silencing via the RNA interference pathway (Fire et al. (1998) Nature 391:806), such siRNA molecules need not be strictly polyribonucleotides, and may instead contain one or more modifications to the nucleic acid to improve its properties as a therapeutic agent. Such agents are occasionally referred to as "siNA" for short interfering nucleic acids. Although such changes may formally move the molecule outside the definition of a "ribo"nucleotide, such molecules are nonetheless referred to as "siRNA" molecules herein. For example, some siRNA duplexes comprise two 19 - 25 nt (e.g. 21 nt) strands that pair to form a 17 - 23 basepair (e.g. 19 base pair) polyribonucleotide duplex with TT (deoxyribonucleotide) 3' overhangs on each strand. Other variants of nucleic acids used to induce gene silencing via the RNA interference pathway include short hairpin RNAs ("shRNA"), for example as disclosed in U.S. Pat. App. Publication No. 2006/0115453

Soluble ST2 is believed to bind to IL-33 in solution, and thus to act as a natural antagonist of IL-33 by blocking binding to the IL-33 receptor of cell surfaces. Chackerian et al. (2007) J Immunol 179:2551-2555; Leung et al. (2004) J. Immunol. 173:145-150. Human sST2 comprises the sequence of amino acid residues 19 - 328 of SEQ ID NO: 8. Use of an sST2-Fc fusion protein in the treatment of lung fibrosis is disclosed at WO 2007/140205.

The broad scope of this invention is best understood with reference to the following examples, which are not intended to limit the inventions to the specific embodiments.

### EXAMPLES

### Example 1

### General Methods

Standard methods in molecular biology are described. Maniatis et al. (1982) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA. Standard methods also appear in Ausbel et al. (2001) Current Protocols in Molecular Biology, Vols. 1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described. Coligan et al. (2000) Current Protocols in Protein Science, Vol. 1, John Wiley and Sons, Inc., New York. Chemical analysis, chemical modification, post-translational modification, production of fusion proteins, glycosylation of proteins are described. *See, e.g.,* Coligan et al. (2000) Current Protocols in Protein Science, Vol. 2, John Wiley and Sons, Inc., New York; Ausubel et al. (2001) Current Protocols in Molecular Biology, Viol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391. Production, purification, and fragmentation of polyclonal and monoclonal antibodies are described. Coligan et al. (2001) Current Protocols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra.* Standard techniques for characterizing ligand/receptor interactions are available. *See, e.g.,* Coligan et al. (2001) Current Protocols in Immunology, Vol. 4, John Wiley, Inc., New York.

Methods for flow cytometry, including fluorescence activated cell sorting detection systems (FACS®), are available. *See, e.g.,* Owens et al. (1994) Flow Cytometry Principles for Clinical Laboratory Practice, John Wiley and Sons, Hoboken, NJ; Givan (2001) Flow Cytometry, 2nd ed.; Wiley-Liss, Hoboken, NJ; Shapiro (2003) Practical Flow Cytometry, John Wiley and Sons, Hoboken, NJ. Fluorescent reagents suitable for modifying nucleic acids, including nucleic acid primers and probes, polypeptides, and antibodies, for use, e.g., as diagnostic reagents, are available. Molecular Probes (2003) Catalog, Molecular Probes, Inc., Eugene, OR; Sigma-Aldrich (2003) Catalog, St. Louis, MO.

Standard methods of histology of the immune system are described. *See, e.g.,* Muller-Harmelink (ed.) (1986) Human Thymus: Histopathology and Pathology, Springer Verlag, New York, NY; Hiatt, et al. (2000) Color Atlas of Histology, Lippincott, Williams, and Wilkins, Phila., PA; Louis, et al. (2002) Basic Histology: Text and Atlas, McGraw-Hill, New York, NY.

Statistical analysis may be performed using commercially available software, including but not limited to JMP® Statistical Discovery Software, SAS Institute Inc., Cary, North Carolina, USA.

Cell growth media and methods are provided, e.g., at Int'l. Pat. Appl. Pub. No. WO 90/03430 and U.S. Pat. No. 5,830,761.

### Example 2

### Methods Specifically Related to the Experiments Described Herein

Animals and animal experiments were as follows. B6/129.IL-1RAcP^{-/-}, B6.IL-1R1^{-/-}, B6.RAG^{-/-} and 129S6.IL-13^{-/-} mice were housed in specific pathogen free conditions at Schering-Plough Biopharma according to IACUC guidelines. Cullinan (1998) J. Immunol. 161:5614-5620; Mombaerts et al. (1992) Cell 68:869-877; Glaccum et al. (1997) J. Immunol. 159:3364-3371; McKenzie et al. (1998) Curr. Biol. 8:339-342. B6.ST2^{-/-} mice were a kind gift from the Neurath lab, University of Mainz; Hoshino et al. (1999) J. Exp. Med. 190:1541-1547.. BALB/c.ΔdblGATA, WB/B6F1.cKit^{w/v}, B6.IL-4^{-/-} and appropriate control mice were obtained from the Jackson laboratories. Yu et al. (2002) J. Exp. Med. 195:1387-1395; Metwali et al. (1996) J. Immunol. 157:4546-4553. B6/129 mice were obtained from the Jackson laboratories. C57B1/6 and 129S6 mice were obtained from Taconic. Age and sex matched mice of 8 to 16 weeks of age were used for the experiments described.

Mice were injected subcutaneously daily for 7 days with 5 µg of MSA or recombinant mIL-33. Recombinant IL-33 (AA112-270) was produced in *E. coli* by Aragen Biosciences and contained < 0.5 EU/ml endotoxin. Twenty-four hours after the final injection the injection site was harvested for analysis. Experimental protocols were approved by the Schering-Plough Biopharma IACUC.

Quantitative RT-PCR was performed as follows. RNA isolation was performed by standard techniques and gene expression was calculated using the Δ-ΔCt method (using the mean cycle threshold value for ubiquitin and the gene of interest for each sample). Primers were obtained commercially from Applied Biosystems (Foster City, CA) or designed using Primer Express (PE Biosystems, Foster City, CA). The equation 1.8e (Ct ubiquitin - Ct gene of interest) x 10⁴ was used to obtain the normalized values.

Histology was performed as follows. Skin samples from the injection site were harvested and formalin fixed for 24 hours. Samples were then paraffin embedded and 5 µm section cut. Sections were stained with H&E, modified Masson's trichrome, or astra blue and violet red according to the manufacturer's directions (American MasterTech). To quantify mast cell and eosinophil skin infiltration, astra blue and violet red stained sections were scanned using a Mirax Midi slide scanner (Zeiss). Mast cells were identified based on the characteristic astra blue staining of mast cell granules and the presence of a singular nucleus whereas eosinophils were identified by violet red cytoplasmic staining co-localized with a bi-lobed nucleus. A minimum of area of 0.5 mm² of skin per mouse was randomly selected and surveyed to obtain manual cell counts. Images were obtained using a brightfield microscope (Olympus Model BX51) with attached digital camera (Q Imaging Model Retiga 2000R) and were white balanced using Adobe Photoshop Elements (v.2.0).

Immunohistochemistry (IHC) was performed as follows. Immunohistochemical studies were performed on formalin-fixed, paraffin-embedded tissue sections using a rabbit polyclonal antibody against anti-human CD3 (Catalog #A0452, Dako Corp., dilution 1:200) and a rat monoclonal antibody against anti-human F4/80 (Clone BM8, eBioscience, dilution 1:400). Paraffin embedded tissues were sectioned at 5 µm thickness, deparaffinized and quenched with 3% hydrogen peroxide for 10 minutes. Slides were heat retrieved with Citrate Buffer at pH 6.1 (Cat. # S1699, Dako Corp.) for 4 minutes at 123 °C using the Biocare Decloaker chamber, then cooled for 15 minutes followed by a running tap water rinse. Slides were mounted on a DAKO Autostainer and covered with fresh TBS to prevent drying of sections. Sections were then incubated with anti-CD3 or anti-F4/80 antibodies at room temperature for 60 minutes and rinsed with TBS. CD3 stained sections were incubated for 30-minute in Rabbit Envision-Plus (Catalog #K4011, Dako Corp.). F4/80 stained sections were incubated for 30 minutes in Rat Probe and then Rat Polymer-HRP (Catalog #RT517L, Biocare Medical). Slides were rinsed with TBS and developed with DAB-Plus (Dako Corp.), counterstained in Modified Mayer's Hematoxylin and blued in 0.3% ammonia water followed by a tap water rinse. CD3⁺ and F4/80⁺ cells were counted as described above.

Collagen was quantified as follows. Total soluble collagen levels in the skin were quantified using the Sircol™ Assay according to the manufacturer's directions (BioColor Ltd). Briefly, a 6 mm dermal punch biopsy was isolated from the injection site and subcutaneous fat removed. Samples were homogenized in 3 ml of 0.5 M acetic acid supplemented with cOmplete™ protease inhibitors (Roche). After overnight extraction at 4 °C, the samples were spun down and 0.2 ml of extract was assayed to determine collagen content. Results are reported as total micrograms of collagen extracted from 6 mm dermal punch biopsies.

Statistical analysis was performed as follows. Statistical analyses to compare collagen content in skin and cell numbers between experimental and control groups were performed using Student's *t* test, with *p* values ≤ 0.05 considered significant. Studies utilizing TAQman analysis to compare gene expression levels between control and experimental groups were analyzed using the Mann-Whitney U-test. *p* values ≤ 0.05 considered significant. Statistical analyses were performed using GraphPad Prism version 4.02 for Windows (GraphPad Software).

### Example 3

### IL-33 Mediated Induction of Skin Fibrosis and Inflammation

To assess the consequences of dysregulated IL-33 release in skin IL-33 or control protein (MSA) was injected subcutaneously every day for 7 days. IL-33 and MSA injected mice did not exhibit any discernible changes in behavior over the course of the injection series (e.g. pruritus) nor was any overt skin pathology observed. Nevertheless, histological analysis of skin from IL-33- but not MSA-injected mice revealed prominent inflammation and edema at the injection site (FIG. 1A) and development of subcuticular fibrosis as evidenced by Masson's Trichrome staining (not shown). Soluble collagen levels were increased approximately 4-fold in skin punch biopsies obtained from IL-33-injected mice (FIG. 1B). The majority of infiltrating leukocytes consisted of granulocytic leucocytes with dense clusters of these cells occasionally observed in subcutaneous adipose tissue (FIG. 1A). Histochemical staining revealed that the majority of infiltrating leucocytes were eosinophils and that subcutaneous injection of IL-33 caused significant accumulation of these cells compared to MSA-injected skin (FIG. 1C). Expression levels of eosinophil major basic protein in skin from IL-33 and control protein treated mice yielded a similar pattern of results (data not shown). CD3⁺ mononuclear cells were also significantly increased in skin obtained from IL-33 injected mice (FIG. 1D) whereas mast cells and B220⁺ cells were present at similar numbers in skin sections obtained from mice injected with IL-33 or MSA (data not shown). No pathological changes were observed in skin sections adjacent to the injection site (data not shown).

### Example 4

### The Role of ST2 and IL-1RAcP in IL-33-Induced Skin Pathology

To determine if IL-33-induced inflammation is dependent on signaling through the IL-33 receptor, which is comprised of ST2 and IL-1RAcP, ST2^{-/-} and IL-1RAcP^{-/-} mice were injected subcutaneously with IL-33. Neither IL-1RAcP^{-/-} nor ST2^{-/-} mice exhibited histological signs of inflammation or subcuticular fibrosis after receiving IL-33 injections (FIG. 2). IL-1RAcP is a shared subunit of the IL-33R that also pairs with IL-1R1 to form the heteromeric receptor for IL-1α and IL-β. Cullinan (1998) J. Immunol. 161:5614-5620. To determine if signaling through IL-1R1 is required for the development of IL-33-induced cutaneous fibrosis and inflammation we treated IL-1R1^{-/-} mice subcutaneously with IL-33. IL-1R1^{-/-} mice injected with IL-33 developed inflammation and subcuticular fibrosis in a manner that was indistinguishable from control mice, indicating that IL-1α/β signaling is not required for IL-33 induced skin pathology (data not shown). Collectively, these results demonstrate that repeated subcutaneous administration of IL-33 induces an ST2/IL-1RAcP-dependent skin inflammation and fibrosis.

### Example 5

### IL-33-Induced Modulation of Th2 and Extracellular Matrix-Associated Gene Expression

Gene expression in skin obtained from IL-33 injected mice was examined. IL-33-injected mice had significantly elevated expression of Th2 cytokines IL-4, IL-5 and IL-13 (FIG. 3A), whereas expression of the Th1- and Th17-associated cytokines, IFN-γ and IL-17 respectively, remained unchanged (data not shown). As repeated injection of IL-33 induced the development of subcuticular fibrosis locally, we also examined expression of a panel of extracellular matrix (ECM) associated genes (FIG. 4). IL-33-injected skin exhibited substantially increased expression of various components of the ECM, such as collagen VIa, collagen IIIa and fibronectin 1 (FIGS. 3B and 4). In addition, expression of ECM-modifying components, such as TIMP-1, MMP-12 and MMP-13 were also significantly elevated (FIG. 3C). Interestingly, IL-33 treatment also resulted in significantly reduced expression of several collagen isoforms, including collagens II, IV, XI, XIII, XVI and XVIII (FIG. 4). Thus, these results highlight the novel observation that IL-33 can modulate expression of ECM-associated genes in the skin.

TGF-β and IL-13 are two cytokines known to play a critical role in promoting fibrotic disease in multiple animal models. Wynn (2008) J. Pathol. 214:199-210. Skin isolated from IL-33 injected mice expressed levels of TGF-β that were similar to those observed in MSA-injected skin samples (FIG. 3D). Because expression of IL-13 (FIG. 3A), but not TGF-β (FIG. 3D), was significantly increased in IL-33 injected skin samples, it appears that IL-33 induced fibrosis may develop in an IL-13 dependent manner.

### Example 6

### The Role of IL-13 in IL-33 Activity

To determine if IL-33 induced skin pathology requires IL-13, IL-13^{-/-} mice were injected with IL-33 subcutaneously daily for 7 days, skin samples were examined histologically (FIG. 5A), and collagen content was quantified. Skin sections prepared from IL-13^{-/-} mice injected with IL-33 revealed only minor trichrome staining in the subcutis (data not shown), and the collagen content in skin obtained from IL-33 injected IL-13^{-/-} mice was found to be significantly reduced compared to wild type animals treated with IL-33 (FIG. 5B). Strikingly, the soluble collagen content in skin obtained from IL-33 injected IL-13^{-/-} mice was similar to levels observed in control protein treated animals, collectively indicating that IL-13 acts as a critical mediator of IL-33-induced skin fibrosis. Expression levels of Collagen VIa, Collagen IIIa, MMP12, MMP13 and TIMP1 were reduced in skin obtained from IL-33 injected IL-13^{-/-} mice compared to wild type mice (FIGS. 5C and 5D), whereas expression of fibronectin was similarly induced by IL-33 in wild type and IL-13^{-/-} mice (FIG. 5E), indicating that IL-33-dependent expression of fibronectin occurs via an IL-13 independent mechanism.

In addition, IL-33-injected IL-13^{-/-} mice exhibited a non-significant trend towards reduced eosinophil infiltrates compared with IL-33-injected wild type mice (FIG. 5F).

Table 1 provides a brief description of the sequences in the sequence listing.

**Table 1**

| Sequence Identifiers | |
|---|---|
| SEQ ID NO: | Description |
| 1 | IL-1RAcP isoform 2 nucleic acid |
| 2 | IL-1RAcP isoform 2 polypeptide |
| 3 | IL-1RAcP isoform 1 nucleic acid |
| 4 | IL-1RAcP isoform 1 polypeptide |
| 5 | IL-33 nucleic acid |
| 6 | IL-33 polypeptide |
| 7 | ST2 isoform 2 nucleic acid |
| 8 | ST2 isoform 2 polypeptide |
| 9 | ST2 isoform 1 nucleic acid |
| 10 | ST2 isoform 1 polypeptide |

## Claims

1. An antagonist of IL-33 for use in treating scleroderma in a subject, wherein said antagonist is selected from:
an antagonistic antibody, or antigen binding fragment thereof, that specifically binds to IL-33 (SEQ ID NO: 6);
a soluble ST2 polypeptide;
an antisense nucleic acid that specifically blocks the production of IL-33 (SEQ ID NO: 6); and
a siRNA that specifically blocks the production of IL-33 (SEQ ID NO: 6).

2. The antagonist for use according to Claim 1, which is a chimeric, humanized or human antibody, or antigen binding fragment thereof, , that specifically binds to IL-33 (SEQ ID NO: 6).

3. The antagonist for use according to Claim 2, wherein the antibody, or antigen binding fragment thereof, is a Fab, Fab', F(ab')₂, or Fv antibody fragment.

4. The antagonist for use according to Claim 1, which is a soluble ST2 polypeptide.

5. The antagonist for use according to Claim 1, which is an antisense nucleic acid that specifically blocks the production of IL-33 (SEQ ID NO: 6).

6. The antagonist for use according to Claim 1, which is an siRNA that specifically blocks the production of IL-33 (SEQ ID NO: 6).

7. The antagonist for use according to Claim 1, which blocks the binding of IL-33 to its receptor, which receptor comprises ST2 and IL-1RAcP.

8. The antagonist for use according to any preceding Claim, wherein the subject is a human, and the IL-33 is human IL-33 (SEQ ID NO: 6).

9. Use of an antagonist of IL-33 as defined in any preceding claim for the manufacture of a medicament for treating scleroderma.

## Patentansprüche

1. Ein IL-33-Antagonist zur Verwendung bei der Behandlung von Sklerodermie bei einem Subjekt, wobei der Antagonist ausgewählt ist aus:
einem antagonistischen Antikörper oder einem antigenbindenden Fragment davon, der/das spezifisch an IL33 (SEQ ID NR.: 6) bindet,
einem löslichen ST2-Polypeptid,
einer Antisense-Nukleinsäure, die spezifisch die Produktion von IL-33 (SEQ ID NR.: 6) blockiert, und
einer siRNA, die spezifisch die Produktion von IL-33 (SEQ ID NR.: 6) blockiert.

2. Der Antagonist zur Verwendung gemäß Anspruch 1, der ein chimärer, humanisierter oder menschlicher Antikörper oder ein antigenbindendes Fragment davon ist, der/das spezifisch an IL-33 (SEQ ID NR.: 6) bindet.

3. Der Antagonist zur Verwendung gemäß Anspruch 2, wobei der Antikörper oder das antigenbindende Fragment davon ein Fab-, Fab'-, F(ab')₂- oder Fv-Antikörper-Fragment ist.

4. Der Antagonist zur Verwendung gemäß Anspruch 1, der ein lösliches ST2-Polypeptid ist.

5. Der Antikörper zur Verwendung gemäß Anspruch 1, der eine Antisense-Nukleinsäure ist, die spezifisch die Produktion von IL-33 (SEQ ID NR.: 6) blockiert.

6. Der Antagonist zur Verwendung gemäß Anspruch 1, der eine siRNA ist, die spezifisch die Produktion von IL-33 (SEQ ID NR.: 6) blockiert.

7. Der Antagonist zur Verwendung gemäß Anspruch 1, der die Bindung von IL-33 an seinen Rezeptor blockiert, wobei der Rezeptor ST2 und IL-1RAcP umfasst.

8. Der Antagonist zur Verwendung gemäß einem vorhergehenden Anspruch, wobei das Subjekt ein Mensch ist und das IL-33 menschliches IL-33 (SEQ ID NR.: 6) ist.

9. Verwendung eines IL-33-Antagonisten wie in einem vorhergehenden Anspruch definiert zur Herstellung eines Medikaments zur Behandlung von Sklerodermie.

## Revendications

1. Antagoniste d'IL-33 pour une utilisation pour le traitement de la sclérodermie chez un sujet, dans lequel ledit antagoniste est sélectionné parmi :
un anticorps antagonistique, ou son fragment de liaison d'antigène, qui est lié de manière spécifique à IL-33 (SEQ ID NO:6) ;
un polypeptide de ST2 soluble ;
un acide nucléique antisens qui bloque de manière spécifique la production d'IL-33 (SEQ ID NO:6) ; et
un petit ARN interférant qui bloque de manière spécifique la production d'IL-33 (SEQ ID NO:6).

2. Antagoniste pour une utilisation selon la revendication 1, lequel est un anticorps chimérique, humanisé ou humain, ou son fragment de liaison d'antigène, qui est lié de manière spécifique à IL-33 (SEQ ID NO:6).

3. Antagoniste pour une utilisation selon la revendication 2, dans lequel l'anticorps, ou son fragment de liaison d'antigène, est un fragment d'anticorps Fab, Fab', F(ab')₂ ou Fv.

4. Antagoniste pour une utilisation selon la revendication 1, lequel est un polypeptide de ST2 soluble.

5. Antagoniste pour une utilisation selon la revendication 1, lequel est un acide nucléique antisens qui bloque de manière spécifique la production d'IL-33 (SEQ ID NO:6).

6. Antagoniste pour une utilisation selon la revendication 1, lequel est un petit ARN interférant qui bloque de manière spécifique la production d'IL-33 (SEQ ID NO:6).

7. Antagoniste pour une utilisation selon la revendication 1, lequel bloque la liaison d'IL-33 sur son récepteur, lequel récepteur comprend du ST2 et de l'IL-1RAcP.

8. Antagoniste pour une utilisation selon l'une quelconque des revendications précédentes, dans lequel le sujet est un être humain et l'IL-33 est de l'IL-33 (SEQ ID NO:6) humain.

9. Utilisation d'un antagoniste d'IL-33 tel que défini selon l'une quelconque des revendications précédentes pour la fabrication d'un médicament pour traiter la sclérodermie.


### SEQUENCE LISTING

<110> Schering Corporation Rankin, Andrew L. Pflanz, Stefan Mumm, John
<120> USE OF IL-33 ANTAGONISTS TO TREAT FIBROTIC DISEASE
<130> BP2009.7052
<150> 61/241,324
   <151> 2009-09-10
<160> 10
<170> PatentIn version 3.5
<210> 1
   <211> 2063
   <212> DNA
   <213> Homo sapiens
<400> 1
   tgccgggatc caggtctccg gggtccgctt tggccagagg cgcggaagga agcagtgccc      60
   ggcgacactg cacccatccc ggctgctttt gctgcgccct ctcagcttcc caagaaaggc     120
   atcgtcatgt gatcatcacc taagaactag aacatcagca ggccctagaa gcctcactct     180
   tgcccctccc tttaatatct caaaggatga cacttctgtg gtgtgtagtg agtctctact     240
   tttatggaat cctgcaaagt gatgcctcag aacgctgcga tgactgggga ctagacacca     300
   tgaggcaaat ccaagtgttt gaagatgagc cagctcgcat caagtgccca ctctttgaac     360
   acttcttgaa attcaactac agcacagccc attcagctgg ccttactctg atctggtatt     420
   ggactaggca ggaccgggac cttgaggagc caattaactt ccgcctcccc gagaaccgca     480
   ttagtaagga gaaagatgtg ctgtggttcc ggcccactct cctcaatgac actggcaact     540
   atacctgcat gttaaggaac actacatatt gcagcaaagt tgcatttccc ttggaagttg     600
   ttcaaaaaga cagctgtttc aattccccca tgaaactccc agtgcataaa ctgtatatag     660
   aatatggcat tcagaggatc acttgtccaa atgtagatgg atattttcct tccagtgtca     720
   aaccgactat cacttggtat atgggctgtt ataaaataca gaattttaat aatgtaatac     780
   ccgaaggtat gaacttgagt ttcctcattg ccttaatttc aaataatgga aattacacat     840
   gtgttgttac atatccagaa aatggacgta cgtttcatct caccaggact ctgactgtaa     900
   aggtagtagg ctctccaaaa aatgcagtgc cccctgtgat ccattcacct aatgatcatg     960
   tggtctatga gaaagaacca ggagaggagc tactcattcc ctgtacggtc tattttagtt    1020
   ttctgatgga ttctcgcaat gaggtttggt ggaccattga tggaaaaaaa cctgatgaca    1080
   tcactattga tgtcaccatt aacgaaagta taagtcatag tagaacagaa gatgaaacaa    1140
   gaactcagat tttgagcatc aagaaagtta cctctgagga tctcaagcgc agctatgtct    1200
   gtcatgctag aagtgccaaa ggcgaagttg ccaaagcagc caaggtgaag cagaaaggta    1260
   atagatgcgg tcagtgatga atctctcagc tccaaattaa cattgtggtg aataaggaca 1320
   aaaggagaga ttgagaacaa gagagctcca gcacctagcc cgacggcatc taacccatag 1380
   taatgaatca aacttaaatg aaaaatatga aagttttcat ctatgtaaga tactcaaaat 1440
   attgtttctg atattgttag taccgtaatg cccaaatgta gctaaaaaaa tcgacgtgag 1500
   tacagtgaga cacaattttg tgtctgtaca attatgaaaa attaaaaaca aagaaaatat 1560
   tcaaagctac caaagataga aaaaactggt agagccacat attgttggtg aattattaag 1620
   acccttttaa aaatcattca tggtagagtt taagagtcat aaaaaagatt gcatcatctg 1680
   acctaagact ttcggaattt ttcctgaaca aataacagaa agggaattat atacctttta 1740
   atattattag aagcattatc tgtagttgta aaacattatt aatagcagcc atccaattgt 1800
   atgcaactaa ttaaggtatt gaatgtttat tttccaaaaa tgcataatta taatattatt 1860
   ttaaacacta tgtatcaata tttaagcagg tttataatat accagcagcc acaattgcta 1920
   aaatgaaaat catttaaatt atgattttaa atggtataaa catgatttct atgttgatag 1980
   tactatatta ttctacaata aatggaaatt ataaagcctt cttgtcagaa gtgctgctcc 2040
   taaaaaaaaa aaaaaaaaaa aaa 2063
<210> 2
   <211> 356
   <212> PRT
   <213> Homo sapiens
<400> 2
   Met Thr Leu Leu Trp Cys Val Val Ser Leu Tyr Phe Tyr Gly Ile Leu
   1               5                   10                  15
   Gln Ser Asp Ala Ser Glu Arg Cys Asp Asp Trp Gly Leu Asp Thr Met
               20                  25                  30
   Arg Gln Ile Gln Val Phe Glu Asp Glu Pro Ala Arg Ile Lys Cys Pro
           35                  40                  45
   Leu Phe Glu His Phe Leu Lys Phe Asn Tyr Ser Thr Ala His Ser Ala
       50                  55                  60
   Gly Leu Thr Leu Ile Trp Tyr Trp Thr Arg Gln Asp Arg Asp Leu Glu
   65                  70                  75                  80
   Glu Pro Ile Asn Phe Arg Leu Pro Glu Asn Arg Ile Ser Lys Glu Lys
                   85                  90                  95
   Asp Val Leu Trp Phe Arg Pro Thr Leu Leu Asn Asp Thr Gly Asn Tyr
               100                 105                 110
   Thr Cys Met Leu Arg Asn Thr Thr Tyr Cys Ser Lys Val Ala Phe Pro
           115                 120                 125
   Leu Glu Val Val Gln Lys Asp Ser Cys Phe Asn Ser Pro Met Lys Leu
       130                 135                 140
   Pro Val His Lys Leu Tyr Ile Glu Tyr Gly Ile Gln Arg Ile Thr Cys
   145                 150                 155                 160
   Pro Asn Val Asp Gly Tyr Phe Pro Ser Ser Val Lys Pro Thr Ile Thr
                   165                 170                 175
   Trp Tyr Met Gly Cys Tyr Lys Ile Gln Asn Phe Asn Asn Val Ile Pro
               180                 185                 190
   Glu Gly Met Asn Leu Ser Phe Leu Ile Ala Leu Ile Ser Asn Asn Gly
           195                 200                 205
   Asn Tyr Thr Cys Val Val Thr Tyr Pro Glu Asn Gly Arg Thr Phe His
       210                 215                 220
   Leu Thr Arg Thr Leu Thr Val Lys Val Val Gly Ser Pro Lys Asn Ala
   225                 230                 235                 240
   Val Pro Pro Val Ile His Ser Pro Asn Asp His Val Val Tyr Glu Lys
                   245                 250                 255
   Glu Pro Gly Glu Glu Leu Leu Ile Pro Cys Thr Val Tyr Phe Ser Phe
               260                 265                 270
   Leu Met Asp Ser Arg Asn Glu Val Trp Trp Thr Ile Asp Gly Lys Lys
           275                 280                 285
   Pro Asp Asp Ile Thr Ile Asp Val Thr Ile Asn Glu Ser Ile Ser His
       290                 295                 300
   Ser Arg Thr Glu Asp Glu Thr Arg Thr Gln Ile Leu Ser Ile Lys Lys
   305                 310                 315                 320
   Val Thr Ser Glu Asp Leu Lys Arg Ser Tyr Val Cys His Ala Arg Ser
                   325                 330                 335
   Ala Lys Gly Glu Val Ala Lys Ala Ala Lys Val Lys Gln Lys Gly Asn
               340                 345                 350
   Arg Cys Gly Gln
           355
<210> 3
   <211> 4726
   <212> DNA
   <213> Homo sapiens
<400> 3
   tgccgggatc caggtctccg gggtccgctt tggccagagg cgcggaagga agcagtgccc      60
   ggcgacactg cacccatccc ggctgctttt gctgcgccct ctcagcttcc caagaaaggc     120
   atcgtcatgt gatcatcacc taagaactag aacatcagca ggccctagaa gcctcactct     180
   tgcccctccc tttaatatct caaaggatga cacttctgtg gtgtgtagtg agtctctact     240
   tttatggaat cctgcaaagt gatgcctcag aacgctgcga tgactgggga ctagacacca     300
   tgaggcaaat ccaagtgttt gaagatgagc cagctcgcat caagtgccca ctctttgaac     360
   acttcttgaa attcaactac agcacagccc attcagctgg ccttactctg atctggtatt     420
   ggactaggca ggaccgggac cttgaggagc caattaactt ccgcctcccc gagaaccgca     480
   ttagtaagga gaaagatgtg ctgtggttcc ggcccactct cctcaatgac actggcaact     540
   atacctgcat gttaaggaac actacatatt gcagcaaagt tgcatttccc ttggaagttg     600
   ttcaaaaaga cagctgtttc aattccccca tgaaactccc agtgcataaa ctgtatatag     660
   aatatggcat tcagaggatc acttgtccaa atgtagatgg atattttcct tccagtgtca     720
   aaccgactat cacttggtat atgggctgtt ataaaataca gaattttaat aatgtaatac     780
   ccgaaggtat gaacttgagt ttcctcattg ccttaatttc aaataatgga aattacacat     840
   gtgttgttac atatccagaa aatggacgta cgtttcatct caccaggact ctgactgtaa     900
   aggtagtagg ctctccaaaa aatgcagtgc cccctgtgat ccattcacct aatgatcatg     960
   tggtctatga gaaagaacca ggagaggagc tactcattcc ctgtacggtc tattttagtt    1020
   ttctgatgga ttctcgcaat gaggtttggt ggaccattga tggaaaaaaa cctgatgaca    1080
   tcactattga tgtcaccatt aacgaaagta taagtcatag tagaacagaa gatgaaacaa    1140
   gaactcagat tttgagcatc aagaaagtta cctctgagga tctcaagcgc agctatgtct    1200
   gtcatgctag aagtgccaaa ggcgaagttg ccaaagcagc caaggtgaag cagaaagtgc    1260
   cagctccaag atacacagtg gaactggctt gtggttttgg agccacagtc ctgctagtgg    1320
   tgattctcat tgttgtttac catgtttact ggctagagat ggtcctattt taccgggctc    1380
   attttggaac agatgaaacc attttagatg gaaaagagta tgatatttat gtatcctatg    1440
   caaggaatgc ggaagaagaa gaatttgtat tactgaccct ccgtggagtt ttggagaatg     1500
   aatttggata caagctgtgc atctttgacc gagacagtct gcctggggga attgtcacag     1560
   atgagacttt gagcttcatt cagaaaagca gacgcctcct ggttgttcta agccccaact    1620
   acgtgctcca gggaacccaa gccctcctgg agctcaaggc tggcctagaa aatatggcct    1680
   ctcggggcaa catcaacgtc attttagtac agtacaaagc tgtgaaggaa acgaaggtga    1740
   aagagctgaa gagggctaag acggtgctca cggtcattaa atggaaaggg gaaaaatcca    1800
   agtatccaca gggcaggttc tggaagcagc tgcaggtggc catgccagtg aagaaaagtc 1860
   ccaggcggtc tagcagtgat gagcagggcc tctcgtattc atctttgaaa aatgtatgaa 1920
   aggaataatg aaaagggtaa aaagaacaag gggtgctcca ggaagaaaga gtccccccag 1980
   tcttcattcg cagtttatgg tttcataggc aaaaataatg gtctaagcct cccaataggg 2040
   ataaatttag ggtgactgtg tggctgacta ttctgcttcc tcaggcaaca ctaaagttta 2100
   gaaagatatc atcaacgttc tgtcaccagt ctctgatgcc actatgttct ttgcaggcaa 2160
   agacttgttc aatgcgaatt tccccttcta cattgtctat ccctgttttt atatgtctcc 2220
   attcttttta aaatcttaac atatggagca gcctttccta tgaatttaaa tatgccttta 2280
   aaataagtca ctgttgacag ggtcatgagt ttccgagtat agttttcttt ttatcttatt 2340
   tttactcgtc cgttgaaaag ataatcaagg cctacatttt agctgaggat aatgaacttt 2400
   tttcctcatt cggctgtata atacataacc acagcaagac tgacatccac ttaggatgat 2460
   acaaagcagt gtaactgaaa atgtttcttt taattgattt aaaggacttg tcttctatac 2520
   cacccttgtc ctcatctcag gtaatttatg aaatctatgt aaacttgaaa aatatttctt 2580
   aatttttgtt tttgctccag tcaattcctg attatccaca ggtcaaccca cattttttca 2640
   ttccttctcc ctatctgctt atatcgcatt gctcatttag agtttgcagg aggctccata 2700
   ctaggttcag tctgaaagaa atctcctaat ggtgctatag agagggaggt aacagaaaga 2760
   ctcttttagg gcatttttct gactcatgaa aagagcacag aaaaggatgt ttggcaattt 2820
   gtcttttaag tcttaacctt gctaatgtga atactgggaa agtgattttt tctcactcgt 2880
   ttttgttgct ccattgtaaa gggcggaggt cagtcttagt ggccttgaga gttgcttttg 2940
   gcattaatat tctaagagaa ttaactgtat ttcctgtcac ctattcacta gtgcaggaaa 3000
   tatacttgct ccaaataagt cagtatgaga agtcactgtc aatgaaagtt gttttgtttg 3060
   ttttcagtaa tattttgctg tttttaagac ttggaaaact aagtgcagag tttacagagt 3120
   ggtaaatatc tatgttacat gtagattata catatatata cacacgtgta tatgagatat 3180
   atatcttata tctccacaaa cacaaattat atatatacat atccacacac atacattaca 3240
   tatatctgtg tatataaatc cacatgcaca tgaaatatat atatatatat aatttgtgtg 3300
   tgtgtatgtg tatgtatatg actttaaata gctatgggta caatattaaa aaccactgga 3360
   actcttgtcc agtttttaaa ttatgttttt actggaatgt ttttgtgtca gtgttttctg 3420
   tacatattat ttgttaattc acagctcaca gagtgatagt tgtcatagtt cttgccttcc 3480
   ctaagtttat ataaataact taagtattgc tacagtttat ctaggttgca gtggcatctg 3540
   ctgtgcacag agcttccatg gtcactgcta agcagtagcc agccatcggg cattaattga 3600
   tttcctacta tattcccagc agacacattt agaaactaag ctatgttaac ctcagtgctc 3660
   aactatttga actgttgagt gataaaggaa acaaatataa ctgtaaatga atcttggtat 3720
   cctgtgaaac agaataattc gtaatttaag aaagccctta tcccggtaac atgaatgttg 3780
   atgaacaaat gtaaaattat atcctatatt taagtaccca taataaatca tttccctcta      3840
   taagtgttat tgattatttt aaattgaaaa aagtttcact tggatgaaaa aagtagaaaa      3900
   gtaggtcatt cttggatcta ctttttttta gccttattaa tatttttccc tattagaaac      3960
   cacaattact ccctctatta acccttcact tactagacca gaaaagaact tattccagat      4020
   aagctttgaa tatcaattct tacataaact ttaggcaaac agggaatagt ctagtcacca      4080
   aaggaccatt ctcttgccaa tgctgcattc cttttgcact tttggattcc atatttatcc      4140
   caaatgctgt tgggcacccc tagaaatacc ttgatgtttt ttctatttat atgcctgcct      4200
   ttggtactta attttacaaa tgctgtaata taaagcatat caagtttatg tgatacgtat      4260
   cattgcaaga gaatttgttt caagattttt ttttaatgtt ccagaagatg gccaatagag      4320
   aacattcaag ggaaatgggg aaacataatt tagagaacaa gaacaaacca tgtctcaaat      4380
   ttttttaaaa aaaattaatg gttttaaata tatgctatag ggacgttcca tgcccaggtt      4440
   aacaaagaac tgtgatatat agagtgtcta attacaaaat catatacgat ttatttaatt      4500
   ctcttctgta ttgtaactta gatgattccc aaggactcta ataaaaaatc acttcattgt      4560
   atttggaaac aaaaacatca ttcattaatt acttattttc tttccatagg ttttaatatt      4620
   ttgagagtgt cttttttatt tcattcatga acttttgtat ttttcatttt tcatttgatt      4680
   tgtaaattta cttatgttaa aaataaacca tttattttca gctttg                     4726
<210> 4
   <211> 570
   <212> PRT
   <213> Homo sapiens
<400> 4
   Met Thr Leu Leu Trp Cys Val Val Ser Leu Tyr Phe Tyr Gly Ile Leu
   1               5                   10                  15
   Gln Ser Asp Ala Ser Glu Arg Cys Asp Asp Trp Gly Leu Asp Thr Met
               20                  25                  30
   Arg Gln Ile Gln Val Phe Glu Asp Glu Pro Ala Arg Ile Lys Cys Pro
           35                  40                  45
   Leu Phe Glu His Phe Leu Lys Phe Asn Tyr Ser Thr Ala His Ser Ala
       50                  55                  60
   Gly Leu Thr Leu Ile Trp Tyr Trp Thr Arg Gln Asp Arg Asp Leu Glu
   65                  70                  75                  80
   Glu Pro Ile Asn Phe Arg Leu Pro Glu Asn Arg Ile Ser Lys Glu Lys
                   85                  90                  95
   Asp Val Leu Trp Phe Arg Pro Thr Leu Leu Asn Asp Thr Gly Asn Tyr
               100                 105                 110
   Thr Cys Met Leu Arg Asn Thr Thr Tyr Cys Ser Lys Val Ala Phe Pro
           115                 120                 125
   Leu Glu Val Val Gln Lys Asp Ser Cys Phe Asn Ser Pro Met Lys Leu
       130                 135                 140
   Pro Val His Lys Leu Tyr Ile Glu Tyr Gly Ile Gln Arg Ile Thr Cys
   145                 150                 155                 160
   Pro Asn Val Asp Gly Tyr Phe Pro Ser Ser Val Lys Pro Thr Ile Thr
                   165                 170                 175
   Trp Tyr Met Gly Cys Tyr Lys Ile Gln Asn Phe Asn Asn Val Ile Pro
               180                 185                 190
   Glu Gly Met Asn Leu Ser Phe Leu Ile Ala Leu Ile Ser Asn Asn Gly
           195                 200                 205
   Asn Tyr Thr Cys Val Val Thr Tyr Pro Glu Asn Gly Arg Thr Phe His
       210                 215                 220
   Leu Thr Arg Thr Leu Thr Val Lys Val Val Gly Ser Pro Lys Asn Ala
   225                 230                 235                 240
   Val Pro Pro Val Ile His Ser Pro Asn Asp His Val Val Tyr Glu Lys
                   245                 250                 255
   Glu Pro Gly Glu Glu Leu Leu Ile Pro Cys Thr Val Tyr Phe Ser Phe
               260                 265                 270
   Leu Met Asp Ser Arg Asn Glu Val Trp Trp Thr Ile Asp Gly Lys Lys
           275                 280                 285
   Pro Asp Asp Ile Thr Ile Asp Val Thr Ile Asn Glu Ser Ile Ser His
       290                 295                 300
   Ser Arg Thr Glu Asp Glu Thr Arg Thr Gln Ile Leu Ser Ile Lys Lys
   305                 310                 315                 320
   Val Thr Ser Glu Asp Leu Lys Arg Ser Tyr Val Cys His Ala Arg Ser
                   325                 330                 335
   Ala Lys Gly Glu Val Ala Lys Ala Ala Lys Val Lys Gln Lys Val Pro
               340                 345                 350
   Ala Pro Arg Tyr Thr Val Glu Leu Ala Cys Gly Phe Gly Ala Thr Val
               355                 360                 365
   Leu Leu Val Val Ile Leu Ile Val Val Tyr His Val Tyr Trp Leu Glu
       370                 375                 380
   Met Val Leu Phe Tyr Arg Ala His Phe Gly Thr Asp Glu Thr Ile Leu
   385                 390                 395                 400
   Asp Gly Lys Glu Tyr Asp Ile Tyr Val Ser Tyr Ala Arg Asn Ala Glu
                   405                 410                 415
   Glu Glu Glu Phe Val Leu Leu Thr Leu Arg Gly Val Leu Glu Asn Glu
               420                 425                 430
   Phe Gly Tyr Lys Leu Cys Ile Phe Asp Arg Asp Ser Leu Pro Gly Gly
           435                 440                 445
   Ile Val Thr Asp Glu Thr Leu Ser Phe Ile Gln Lys Ser Arg Arg Leu
       450                 455                 460
   Leu Val Val Leu Ser Pro Asn Tyr Val Leu Gln Gly Thr Gln Ala Leu
   465                 470                 475                 480
   Leu Glu Leu Lys Ala Gly Leu Glu Asn Met Ala Ser Arg Gly Asn Ile
                   485                 490                 495
   Asn Val Ile Leu Val Gln Tyr Lys Ala Val Lys Glu Thr Lys Val Lys
               500                 505                 510
   Glu Leu Lys Arg Ala Lys Thr Val Leu Thr Val Ile Lys Trp Lys Gly
           515                 520                 525
   Glu Lys Ser Lys Tyr Pro Gln Gly Arg Phe Trp Lys Gln Leu Gln Val
       530                 535                 540
   Ala Met Pro Val Lys Lys Ser Pro Arg Arg Ser Ser Ser Asp Glu Gln
   545                 550                 555                 560
   Gly Leu Ser Tyr Ser Ser Leu Lys Asn Val
                   565                 570
<210> 5
   <211> 2644
   <212> DNA
   <213> Homo sapiens
<400> 5
   caacagaata ctgaaaaatg aagcctaaaa tgaagtattc aaccaacaaa atttccacag 60
   caaagtggaa gaacacagca agcaaagcct tgtgtttcaa gctgggaaaa tcccaacaga 120
   aggccaaaga agtttgcccc atgtacttta tgaagctccg ctctggcctt atgataaaaa 180
   aggaggcctg ttactttagg agagaaacca ccaaaaggcc ttcactgaaa acaggtagaa 240
   agcacaaaag acatctggta ctcgctgcct gtcaacagca gtctactgtg gagtgctttg 300
   cctttggtat atcaggggtc cagaaatata ctagagcact tcatgattca agtatcacag 360
   gaatttcacc tattacagag tatcttgctt ctctaagcac atacaatgat caatccatta 420
   cttttgcttt ggaggatgaa agttatgaga tatatgttga agacttgaaa aaagatgaaa 480
   agaaagataa ggtgttactg agttactatg agtctcaaca cccctcaaat gaatcaggtg 540
   acggtgttga tggtaagatg ttaatggtaa ccctgagtcc tacaaaagac ttctggttgc 600
   atgccaacaa caaggaacac tctgtggagc tccataagtg tgaaaaacca ctgccagacc 660
   aggccttctt tgtccttcat aatatgcact ccaactgtgt ttcatttgaa tgcaagactg 720
   atcctggagt gtttataggt gtaaaggata atcatcttgc tctgattaaa gtagactctt 780
   ctgagaattt gtgtactgaa aatatcttgt ttaagctctc tgaaacttag ttgatggaaa 840
   cctgtgagtc ttgggttgag tacccaaatg ctaccactgg agaaggaatg agagataaag 900
   aaagagacag gtgacatcta agggaaatga agagtgctta gcatgtgtgg aatgttttcc 960
   atattatgta taaaaatatt ttttctaatc ctccagttat tcttttattt ccctctgtat 1020
   aactgcatct tcaatacaag tatcagtata ttaaataggg tattggtaaa gaaacggtca 1080
   acattctaaa gagatacagt ctgaccttta cttttctcta gtttcagtcc agaaagaact 1140
   tcatatttag agctaaggcc actgaggaaa gagccatagc ttaagtctct atgtagacag 1200
   ggatccattt taaagagcta cttagagaaa taattttcca cagttccaaa cgataggctc 1260
   aaacactaga gctgctagta aaaagaagac cagatgcttc acagaattat cattttttca 1320
   actggaataa aacaccaggt ttgtttgtag atgtcttagg caacactcag agcagatctc 1380
   ccttactgtc aggggatatg gaacttcaaa ggcccacatg gcaagccagg taacataaat 1440
   gtgtgaaaaa gtaaagataa ctaaaaaatt tagaaaaata aatccagtat ttgtaaagtg 1500
   aataacttca tttctaattg tttaattttt aaaattctga tttttatata ttgagtttaa 1560
   gcaaggcatt cttacacgag gaagtgaagt aaattttagt tcagacataa aatttcactt 1620
   attaggaata tgtaacatgc taaaactttt ttttttttaa agagtactga gtcacaacat 1680
   gttttagagc atccaagtac catataatcc aactatcatg gtaaggccag aaatcttcta 1740
   acctaccaga gcctagatga gacaccgaat taacattaaa atttcagtaa ctgactgtcc 1800
   ctcatgtcca tggcctacca tcccttctga ccctggcttc cagggaccta tgtcttttaa 1860
   tactcactgt cacattgggc aaagttgctt ctaatcctta tttcccatgt gcacaagtct 1920
   ttttgtattc cagcttcctg ataacactgc ttactgtgga atattcattt gacatctgtc 1980
   tcttttcatt tcttttaact accatgccct tgatatatct tttgcacctg ctgaacttca 2040
   tttctgtatc acctgacctc tggatgccaa aacgtttatt ctgctttgtc tgttgtagaa 2100
   ttttagataa agctattaat ggcaatattt ttttgctaaa cgtttttgtt ttttactgtc     2160
   actagggcaa taaaatttat actcaaccat ataataacat tttttaacta ctaaaggagt     2220
   agtttttatt ttaaagtctt agcaatttct attacaactt ttcttagact taacacttat     2280
   gataaatgac taacatagta acagaatctt tatgaaatat gaccttttct gaaaatacat     2340
   acttttacat ttctacttta ttgagaccta ttagatgtaa gtgctagtag aatataagat     2400
   aaaagaggct gagaattacc atacaagggt attacaactg taaaacaatt tatctttgtt     2460
   tcattgttct gtcaataatt gttaccaaag agataaaaat aaaagcagaa tgtatatcat     2520
   cccatctgaa aaacactaat tattgacatg tgcatctgta caataaactt aaaatgatta     2580
   ttaaataatc aaatatatct actacattgt ttatattatt gaataaagta tattttccaa     2640
   atgt                                                                  2644
<210> 6
   <211> 270
   <212> PRT
   <213> Homo sapiens
<400> 6
   Met Lys Pro Lys Met Lys Tyr Ser Thr Asn Lys Ile Ser Thr Ala Lys
   1               5                   10                  15
   Trp Lys Asn Thr Ala Ser Lys Ala Leu Cys Phe Lys Leu Gly Lys Ser
               20                  25                  30
   Gln Gln Lys Ala Lys Glu Val Cys Pro Met Tyr Phe Met Lys Leu Arg
           35                  40                  45
   Ser Gly Leu Met Ile Lys Lys Glu Ala Cys Tyr Phe Arg Arg Glu Thr
       50                  55                  60
   Thr Lys Arg Pro Ser Leu Lys Thr Gly Arg Lys His Lys Arg His Leu
   65                  70                  75                  80
   Val Leu Ala Ala Cys Gln Gln Gln Ser Thr Val Glu Cys Phe Ala Phe
                   85                  90                  95
   Gly Ile Ser Gly Val Gln Lys Tyr Thr Arg Ala Leu His Asp Ser Ser
               100                 105                 110
   Ile Thr Gly Ile Ser Pro Ile Thr Glu Tyr Leu Ala Ser Leu Ser Thr
           115                 120                 125
   Tyr Asn Asp Gln Ser Ile Thr Phe Ala Leu Glu Asp Glu Ser Tyr Glu
       130                 135                 140
   Ile Tyr Val Glu Asp Leu Lys Lys Asp Glu Lys Lys Asp Lys Val Leu
   145                 150                 155                 160
   Leu Ser Tyr Tyr Glu Ser Gln His Pro Ser Asn Glu Ser Gly Asp Gly
                   165                 170                 175
   Val Asp Gly Lys Met Leu Met Val Thr Leu Ser Pro Thr Lys Asp Phe
               180                 185                 190
   Trp Leu His Ala Asn Asn Lys Glu His Ser Val Glu Leu His Lys Cys
           195                 200                 205
   Glu Lys Pro Leu Pro Asp Gln Ala Phe Phe Val Leu His Asn Met His
       210                 215                 220
   Ser Asn Cys Val Ser Phe Glu Cys Lys Thr Asp Pro Gly Val Phe Ile
   225                 230                 235                 240
   Gly Val Lys Asp Asn His Leu Ala Leu Ile Lys Val Asp Ser Ser Glu
                   245                 250                 255
   Asn Leu Cys Thr Glu Asn Ile Leu Phe Lys Leu Ser Glu Thr
               260                 265                 270
<210> 7
   <211> 2542
   <212> DNA
   <213> Homo sapiens
<400> 7
   gaggagggac ctacaaagac tggaaactat tcttagctcc gtcactgact ccaagttcat      60
   cccctctgtc tttcagtttg gttgagatat aggctactct tcccaactca gtcttgaaga     120
   gtatcaccaa ctgcctcatg tgtggtgacc ttcactgtcg tatgccagtg actcatctgg     180
   agtaatctca acaacgagtt accaatactt gctcttgatt gataaacaga atggggtttt     240
   ggatcttagc aattctcaca attctcatgt attccacagc agcaaagttt agtaaacaat     300
   catggggcct ggaaaatgag gctttaattg taagatgtcc tagacaagga aaacctagtt     360
   acaccgtgga ttggtattac tcacaaacaa acaaaagtat tcccactcag gaaagaaatc     420
   gtgtgtttgc ctcaggccaa cttctgaagt ttctaccagc tgcagttgct gattctggta     480
   tttatacctg tattgtcaga agtcccacat tcaataggac tggatatgcg aatgtcacca     540
   tatataaaaa acaatcagat tgcaatgttc cagattattt gatgtattca acagtatctg     600
   gatcagaaaa aaattccaaa atttattgtc ctaccattga cctctacaac tggacagcac     660
   ctcttgagtg gtttaagaat tgtcaggctc ttcaaggatc aaggtacagg gcgcacaagt     720
   catttttggt cattgataat gtgatgactg aggacgcagg tgattacacc tgtaaattta     780
   tacacaatga aaatggagcc aattatagtg tgacggcgac caggtccttc acggtcaagg     840
   atgagcaagg cttttctctg tttccagtaa tcggagcccc tgcacaaaat gaaataaagg      900
   aagtggaaat tggaaaaaac gcaaacctaa cttgctctgc ttgttttgga aaaggcactc      960
   agttcttggc tgccgtcctg tggcagctta atggaacaaa aattacagac tttggtgaac     1020
   caagaattca acaagaggaa gggcaaaatc aaagtttcag caatgggctg gcttgtctag     1080
   acatggtttt aagaatagct gacgtgaagg aagaggattt attgctgcag tacgactgtc     1140
   tggccctgaa tttgcatggc ttgagaaggc acaccgtaag actaagtagg aaaaatccaa     1200
   gtaaggagtg tttctgagac tttgatcacc tgaactttct ctagcaagtg taagcagaat     1260
   ggagtgtggt tccaagagat ccatcaagac aatgggaatg gcctgtgcca taaaatgtgc     1320
   ttctcttctt cgggatgttg tttgctgtct gatctttgta gactgttcct gtttgctggg     1380
   agcttctctg ctgcttaaat tgttcgtcct cccccactcc ctcctatcgt tggtttgtct     1440
   agaacactca gctgcttctt tggtcatcct tgttttctaa ctttatgaac tccctctgtg     1500
   tcactgtatg tgaaaggaaa tgcaccaaca accgtaaact gaacgtgttc ttttgtgctc     1560
   ttttataact tgcattacat gttgtaagca tggtccgttc tatacctttt tctggtcata     1620
   atgaacactc attttgttag cgagggtggt aaagtgaaca aaaaggggaa gtatcaaact     1680
   actgccattt cagtgagaaa atcctaggtg ctactttata ataagacatt tgttaggcca     1740
   ttcttgcatt gatataaaga aatacctgag actgggtgat ttatatgaaa agaggtttaa     1800
   ttggctcaca gttctgcagg ctgtatggga agcatggcgg catctgcttc tggggacacc     1860
   tcaggagctt tactcatggc agaaggcaaa gcaaaggcag gcacttcaca cagtaaaagc     1920
   aggagcgaga gagaggtgcc acactgaaac agccagatct catgagaagt cactcactat     1980
   tgcaaggaca gcatcaaaga gatggtgcta aaccattcat gatgaactca cccccatgat     2040
   ccaatcacct cccaccaggc tccacctcga atactgggga ttaccattca gcatgagatt     2100
   tgggcaggaa cacagaccca aaccatacca cacacattat cattgttaaa ctttgtaaag     2160
   tatttaaggt acatggaaca cacgggaagt ctggtagctc agcccatttc tttattgcat     2220
   ctgttattca ccatgtaatt caggtaccac gtattccagg gagcctttct tggccctcag     2280
   tttgcagtat acacactttc caagtactct tgtagcatcc tgtttgtatc atagcactgg     2340
   tcacattgcc ttacctaaat ctgtttgaca gtctgctcaa cacgactgca agctccatga     2400
   gggcagggac atcatctctt ccatctttgg gtccttagtg caatacctgg cagctagcca     2460
   gtgctcagct aaatatttgt tgactgaata aatgaatgca caaccaaaaa aaaaaaaaaa     2520
   aaaaaaaaaa aaaaaaaaaa aa                                              2542
<210> 8
   <211> 328
   <212> PRT
   <213> Homo sapiens
<220>
   <221> SIGNAL
   <222> (1)..(18)
<220>
   <221> CHAIN
   <222> (19)..(328)
<400> 8
   Met Gly Phe Trp Ile Leu Ala Ile Leu Thr Ile Leu Met Tyr Ser Thr
   1               5                   10                  15
   Ala Ala Lys Phe Ser Lys Gln Ser Trp Gly Leu Glu Asn Glu Ala Leu
               20                  25                  30
   Ile Val Arg Cys Pro Arg Gln Gly Lys Pro Ser Tyr Thr Val Asp Trp
           35                  40                  45
   Tyr Tyr Ser Gln Thr Asn Lys Ser Ile Pro Thr Gln Glu Arg Asn Arg
       50                  55                  60
   Val Phe Ala Ser Gly Gln Leu Leu Lys Phe Leu Pro Ala Ala Val Ala
   65                  70                  75                  80
   Asp Ser Gly Ile Tyr Thr Cys Ile Val Arg Ser Pro Thr Phe Asn Arg
                   85                  90                  95
   Thr Gly Tyr Ala Asn Val Thr Ile Tyr Lys Lys Gln Ser Asp Cys Asn
               100                 105                 110
   Val Pro Asp Tyr Leu Met Tyr Ser Thr Val Ser Gly Ser Glu Lys Asn
           115                 120                 125
   Ser Lys Ile Tyr Cys Pro Thr Ile Asp Leu Tyr Asn Trp Thr Ala Pro
       130                 135                 140
   Leu Glu Trp Phe Lys Asn Cys Gln Ala Leu Gln Gly Ser Arg Tyr Arg
   145                 150                 155                 160
   Ala His Lys Ser Phe Leu Val Ile Asp Asn Val Met Thr Glu Asp Ala
                   165                 170                 175
   Gly Asp Tyr Thr Cys Lys Phe Ile His Asn Glu Asn Gly Ala Asn Tyr
               180                 185                 190
   Ser Val Thr Ala Thr Arg Ser Phe Thr Val Lys Asp Glu Gln Gly Phe
           195                 200                 205
   Ser Leu Phe Pro Val Ile Gly Ala Pro Ala Gln Asn Glu Ile Lys Glu
       210                 215                 220
   Val Glu Ile Gly Lys Asn Ala Asn Leu Thr Cys Ser Ala Cys Phe Gly
   225                 230                 235                 240
   Lys Gly Thr Gln Phe Leu Ala Ala Val Leu Trp Gln Leu Asn Gly Thr
                   245                 250                 255
   Lys Ile Thr Asp Phe Gly Glu Pro Arg Ile Gln Gln Glu Glu Gly Gln
               260                 265                 270
   Asn Gln Ser Phe Ser Asn Gly Leu Ala Cys Leu Asp Met Val Leu Arg
           275                 280                 285
   Ile Ala Asp Val Lys Glu Glu Asp Leu Leu Leu Gln Tyr Asp Cys Leu
       290                 295                 300
   Ala Leu Asn Leu His Gly Leu Arg Arg His Thr Val Arg Leu Ser Arg
   305                 310                 315                 320
   Lys Asn Pro Ser Lys Glu Cys Phe
                   325
<210> 9
   <211> 2058
   <212> DNA
   <213> Homo sapiens
<400> 9
   aaagagaggc tggctgttgt atttagtaaa gctataaagc tgtaagagaa attggctttc      60
   tgagttgtga aactgtgggc agaaagttga ggaagaaaga actcaagtac aacccaatga     120
   ggttgagata taggctactc ttcccaactc agtcttgaag agtatcacca actgcctcat     180
   gtgtggtgac cttcactgtc gtatgccagt gactcatctg gagtaatctc aacaacgagt     240
   taccaatact tgctcttgat tgataaacag aatggggttt tggatcttag caattctcac     300
   aattctcatg tattccacag cagcaaagtt tagtaaacaa tcatggggcc tggaaaatga     360
   ggctttaatt gtaagatgtc ctagacaagg aaaacctagt tacaccgtgg attggtatta     420
   ctcacaaaca aacaaaagta ttcccactca ggaaagaaat cgtgtgtttg cctcaggcca     480
   acttctgaag tttctaccag ctgcagttgc tgattctggt atttatacct gtattgtcag     540
   aagtcccaca ttcaatagga ctggatatgc gaatgtcacc atatataaaa aacaatcaga     600
   ttgcaatgtt ccagattatt tgatgtattc aacagtatct ggatcagaaa aaaattccaa     660
   aatttattgt cctaccattg acctctacaa ctggacagca cctcttgagt ggtttaagaa     720
   ttgtcaggct cttcaaggat caaggtacag ggcgcacaag tcatttttgg tcattgataa     780
   tgtgatgact gaggacgcag gtgattacac ctgtaaattt atacacaatg aaaatggagc     840
   caattatagt gtgacggcga ccaggtcctt cacggtcaag gatgagcaag gcttttctct     900
   gtttccagta atcggagccc ctgcacaaaa tgaaataaag gaagtggaaa ttggaaaaaa      960
   cgcaaaccta acttgctctg cttgttttgg aaaaggcact cagttcttgg ctgccgtcct     1020
   gtggcagctt aatggaacaa aaattacaga ctttggtgaa ccaagaattc aacaagagga     1080
   agggcaaaat caaagtttca gcaatgggct ggcttgtcta gacatggttt taagaatagc     1140
   tgacgtgaag gaagaggatt tattgctgca gtacgactgt ctggccctga atttgcatgg     1200
   cttgagaagg cacaccgtaa gactaagtag gaaaaatcca attgatcatc atagcatcta     1260
   ctgcataatt gcagtatgta gtgtattttt aatgctaatc aatgtcctgg ttatcatcct     1320
   aaaaatgttc tggattgagg ccactctgct ctggagagac atagctaaac cttacaagac     1380
   taggaatgat ggaaagctct atgatgctta tgttgtctac ccacggaact acaaatccag     1440
   tacagatggg gccagtcgtg tagagcactt tgttcaccag attctgcctg atgttcttga     1500
   aaataaatgt ggctatacct tatgcattta tgggagagat atgctacctg gagaagatgt     1560
   agtcactgca gtggaaacca acatacgaaa gagcaggcgg cacattttca tcctgacccc     1620
   tcagatcact cacaataagg agtttgccta cgagcaggag gttgccctgc actgtgccct     1680
   catccagaac gacgccaagg tgatacttat tgagatggag gctctgagcg agctggacat     1740
   gctgcaggct gaggcgcttc aggactccct ccagcatctt atgaaagtac aggggaccat     1800
   caagtggagg gaggaccaca ttgccaataa aaggtccctg aattctaaat tctggaagca     1860
   cgtgaggtac caaatgcctg tgccaagcaa aattcccaga aaggcctcta gtttgactcc     1920
   cttggctgcc cagaagcaat agtgcctgct gtgatgtgca aaggcatctg agtttgaagc     1980
   tttcctgact tctcctagct ggcttatgcc cctgcactga agtgtgagga gcaggaatat     2040
   taaagggatt caggcctc                                                   2058
<210> 10
   <211> 556
   <212> PRT
   <213> Homo sapiens
<220>
   <221> SIGNAL
   <222> (1)..(18)
<220>
   <221> CHAIN
   <222> (19) .. (556)
<400> 10
   Met Gly Phe Trp Ile Leu Ala Ile Leu Thr Ile Leu Met Tyr Ser Thr
   1               5                   10                  15
   Ala Ala Lys Phe Ser Lys Gln Ser Trp Gly Leu Glu Asn Glu Ala Leu
               20                  25                  30
   Ile Val Arg Cys Pro Arg Gln Gly Lys Pro Ser Tyr Thr Val Asp Trp
           35                  40                  45
   Tyr Tyr Ser Gln Thr Asn Lys Ser Ile Pro Thr Gln Glu Arg Asn Arg
       50                  55                  60
   Val Phe Ala Ser Gly Gln Leu Leu Lys Phe Leu Pro Ala Ala Val Ala
   65                  70                  75                  80
   Asp Ser Gly Ile Tyr Thr Cys Ile Val Arg Ser Pro Thr Phe Asn Arg
                   85                  90                  95
   Thr Gly Tyr Ala Asn Val Thr Ile Tyr Lys Lys Gln Ser Asp Cys Asn
               100                 105                 110
   Val Pro Asp Tyr Leu Met Tyr Ser Thr Val Ser Gly Ser Glu Lys Asn
           115                 120                 125
   Ser Lys Ile Tyr Cys Pro Thr Ile Asp Leu Tyr Asn Trp Thr Ala Pro
       130                 135                 140
   Leu Glu Trp Phe Lys Asn Cys Gln Ala Leu Gln Gly Ser Arg Tyr Arg
   145                 150                 155                 160
   Ala His Lys Ser Phe Leu Val Ile Asp Asn Val Met Thr Glu Asp Ala
                   165                 170                 175
   Gly Asp Tyr Thr Cys Lys Phe Ile His Asn Glu Asn Gly Ala Asn Tyr
               180                 185                 190
   Ser Val Thr Ala Thr Arg Ser Phe Thr Val Lys Asp Glu Gln Gly Phe
           195                 200                 205
   Ser Leu Phe Pro Val Ile Gly Ala Pro Ala Gln Asn Glu Ile Lys Glu
       210                 215                 220
   Val Glu Ile Gly Lys Asn Ala Asn Leu Thr Cys Ser Ala Cys Phe Gly
   225                 230                 235                 240
   Lys Gly Thr Gln Phe Leu Ala Ala Val Leu Trp Gln Leu Asn Gly Thr
                   245                 250                 255
   Lys Ile Thr Asp Phe Gly Glu Pro Arg Ile Gln Gln Glu Glu Gly Gln
               260                 265                 270
   Asn Gln Ser Phe Ser Asn Gly Leu Ala Cys Leu Asp Met Val Leu Arg
           275                 280                 285
   Ile Ala Asp Val Lys Glu Glu Asp Leu Leu Leu Gln Tyr Asp Cys Leu
       290                 295                 300
   Ala Leu Asn Leu His Gly Leu Arg Arg His Thr Val Arg Leu Ser Arg
   305                 310                 315                 320
   Lys Asn Pro Ile Asp His His Ser Ile Tyr Cys Ile Ile Ala Val Cys
                   325                 330                 335
   Ser Val Phe Leu Met Leu Ile Asn Val Leu Val Ile Ile Leu Lys Met
               340                 345                 350
   Phe Trp Ile Glu Ala Thr Leu Leu Trp Arg Asp Ile Ala Lys Pro Tyr
           355                 360                 365
   Lys Thr Arg Asn Asp Gly Lys Leu Tyr Asp Ala Tyr Val Val Tyr Pro
       370                 375                 380
   Arg Asn Tyr Lys Ser Ser Thr Asp Gly Ala Ser Arg Val Glu His Phe
   385                 390                 395                 400
   Val His Gln Ile Leu Pro Asp Val Leu Glu Asn Lys Cys Gly Tyr Thr
                   405                 410                 415
   Leu Cys Ile Tyr Gly Arg Asp Met Leu Pro Gly Glu Asp Val Val Thr
               420                 425                 430
   Ala Val Glu Thr Asn Ile Arg Lys Ser Arg Arg His Ile Phe Ile Leu
           435                 440                 445
   Thr Pro Gln Ile Thr His Asn Lys Glu Phe Ala Tyr Glu Gln Glu Val
       450                 455                 460
   Ala Leu His Cys Ala Leu Ile Gln Asn Asp Ala Lys Val Ile Leu Ile
   465                 470                 475                 480
   Glu Met Glu Ala Leu Ser Glu Leu Asp Met Leu Gln Ala Glu Ala Leu
                   485                 490                 495
   Gln Asp Ser Leu Gln His Leu Met Lys Val Gln Gly Thr Ile Lys Trp
               500                 505                 510
   Arg Glu Asp His Ile Ala Asn Lys Arg Ser Leu Asn Ser Lys Phe Trp
           515                 520                 525
   Lys His Val Arg Tyr Gln Met Pro Val Pro Ser Lys Ile Pro Arg Lys
       530                 535                 540
   Ala Ser Ser Leu Thr Pro Leu Ala Ala Gln Lys Gln
   545 550 555